(19) Europäisches Patentamt
European Patent Office
Office européen des brevets

(11) EP 4 491 171 A1

(12) **EUROPEAN PATENT APPLICATION**
published in accordance with Art. 153(4) EPC

(43) Date of publication:
**15.01.2025 Bulletin 2025/03**

(21) Application number: **22937548.0**

(22) Date of filing: **27.04.2022**

(51) International Patent Classification (IPC):
***A61K 9/16*** *(2006.01)* ***A61K 31/365*** *(2006.01)*
***A61K 9/00*** *(2006.01)* ***A61P 33/10*** *(2006.01)*

(52) Cooperative Patent Classification (CPC):
**A61K 9/00; A61K 9/16; A61K 31/365; A61P 33/10;**
Y02A 50/30

(86) International application number:
**PCT/KR2022/005968**

(87) International publication number:
**WO 2023/200035 (19.10.2023 Gazette 2023/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.04.2022 KR 20220045772**

(71) Applicant: **Inventage Lab Inc.**
**Gyeonggi-do 13403 (KR)**

(72) Inventors:
- **KIM, Ju Hee**
  **Seongnam-si, Gyeonggi-do 13494 (KR)**
- **LEE, Sang No**
  **Seongnam-si, Gyeonggi-do 13494 (KR)**

(74) Representative: **BCKIP Part mbB**
**MK1**
**Landsbergerstraße 98, 3.Stock**
**80339 München (DE)**

# (54) MICROPARTICLES CONTAINING MOXIDECTIN AND SUSTAINED-RELEASE INJECTION COMPOSITION CONTAINING SAME

(57) The present invention relates to microparticles containing moxidectin, and a preparation method thereof. Unlike conventional heartworm prevention drugs that have a short half-life and thus require daily or monthly administration, moxidectin is continuously released for at least three months and thus the effect of preventing heartworms can be maintained for at least three months when the microparticles containing moxidectin are administered. In addition, microparticles which have a constant average diameter and a narrow diameter distribution are prepared so that initial over-release of moxidectin is prevented when the microparticles are administered, the release of the drug can be controlled to keep constant the concentration of moxidectin that is valid for at least three months, and foreign body sensation and pain can be reduced when the microparticles are applied in the form of an injection.

FIG. 1

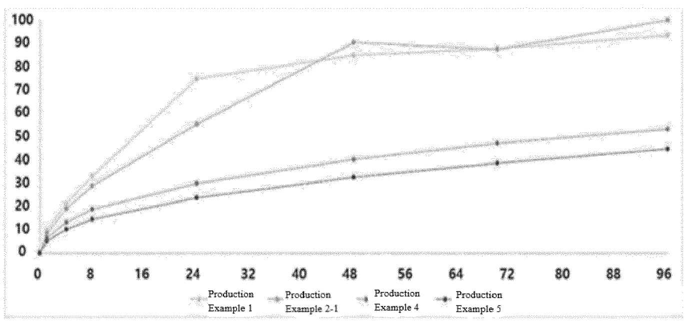


Processed by Luminess, 75001 PARIS (FR)

EP 4 491 171 A1

## Description

### Technical Field

The present invention relates to microparticles containing moxidectin and a sustained-release injectable composition containing the same.

### Background Art

Heartworm disease (HWD) is caused by a parasite worm called *Dirofilaria immitis* that is spread by mosquitoes and affects dogs, cats, and weasels. As the name suggests, heartworms live in the heart of mammals.

Adult heartworms grow up to 30 cm and mainly live in the pulmonary artery and right ventricle. Mature female and male heartworms produce very small larvae called microfilariae (L1). These larvae live in the blood of infected animals and infect other animals through mosquitoes. Two weeks after L1 enters the body of a mosquito, L1 have infection ability, and the larvae having infection ability are transmitted to other animals through the mosquito. The larvae that infected other animals migrate to the pulmonary artery after 3 to 4 months of growth through several stages. Such mature adult heartworms survive on average for 5 to 7 years, and female and male heartworms produce numerous larvae through reproduction.

1 to 200 heartworms may live in the heart and pulmonary arteries of animals infected therewith. Infection results in thickening of the pulmonary artery and inflammation, the heart needs to do more work in order to avoid heartworms and pump blood to the lungs. In addition, inflammation also occurs in the lungs. When the number of infecting heartworms is small, there may be no specific symptoms, but animals infected with heartworms may typically show early symptoms such as avoidance of exercise, coughing, weight loss, and the like. If the infection is severe, symptoms such as severe cough, shortness of breath, and heart failure may appear. If heartworm-infected animals show these symptoms, they may die of heart failure.

When infection with heartworms is confirmed through diagnosis, adult heartworms may be killed with an arsenic-based drug (caparsolate), or treatment may be performed using melarsomine. However, all of the above therapeutic agents have side effects that cause severe irritation at an injection site and damage the liver and the kidneys to some extent.

Accordingly, it is economical and safe to prevent heartworm disease before infection. Prevention is carried out 6 to 8 weeks after birth. Heartwormpreventive drugs include diethylcarbamazine (DEC), which is administered daily, and ivermectin, milbemycin, moxidectin, selamectin and the like, which are administered monthly. The preventive drugs all exhibit excellent preventive effects when administered correctly, but need to be taken daily or monthly, and thus animals may be exposed to the risk of heartworm infection even when skipping administration several times by mistake.

Therefore, there is an urgent need for the development of a heartworm preventive drug which comprises moxidectin capable of preventing heartworm infection and has improved administration convenience by maintaining the efficacy over 3 months or more once administered.

(Prior Art Documents)

(Patent Documents)

(Patent Document 1) KR10-2006-0005472 A1

## DISCLOSURE

### Technical Problem

An object of the present invention is to provide microparticles containing moxidectin and a sustained-release injectable composition containing the same.

Another object of the present invention is to provide microparticles containing moxidectin, which may exhibit a heartworm infection preventive effect over three months or more by releasing moxidectin sustainably over three months or more after administration, unlike conventional heartworm preventive drugs which need to be administered regularly every month due to their short half-life.

Still another object of the present invention is to provide a sustained-release injectable composition containing moxidectin, which may prevent excessive release of moxidectin at an initial stage after administration, maintain the effective concentration of moxidectin at a constant level over 3 months or more by controlling the release of the drug, and reduce foreign body sensation and pain when applied by injection.

## Technical Solution

In order to achieve the above objects, the present invention provides microparticles containing moxidectin and a biodegradable polymer, wherein the biodegradable polymer has an intrinsic viscosity of 0.1 dl/g to 1 dl/g, and the microparticles have an average diameter of 60 to 110 μm.
The microparticles may be spherical in shape, and moxidectin may be distributed uniformly in the microparticles.
The microparticles may have a coefficient of variation (CV) of 5% to 20%.
The microparticles may contain the biodegradable polymer and moxidectin at a weight ratio of 2:1 to 12:1.
The microparticles release moxidectin sustainably over 3 months or more.
The biodegradable polymer may be selected from the group consisting of polylactide (PLA), polylactide-co-glycolide (PLGA), polyphosphazene, polyiminocarbonate, polyphosphoester, polyanhydride, polyorthoester, polycaprolactone, polyhydroxyvalerate, polyhydroxybutyrate, polyamino acids, and mixtures thereof.
The microparticles may have a value of 0.3 to 3 as calculated by the following Equation 1, which represents the plasma concentration of moxidectin measured after mixing the microparticles containing moxidectin with a suspending solution to obtain an injectable formulation and administering the injectable formulation to a plurality of beagle dogs:

[Equation 1]

$$C_{max\text{-}peak\ n}/C_{max\text{—}peak\ n+1}$$

wherein

$C_{max\text{-}peak\ n}$ is an $n^{th}$ Cmax value after administration of the injectable formulation, and
$C_{max\text{-}peak\ n+1}$ is an $n+1^{st}$ Cmax value measured when the plasma concentration of moxidectin increases again after the $n^{th}$ Cmax value.

A sustained-release injectable composition containing moxidectin according to another embodiment of the present invention may : the microparticles containing moxidectin; and a suspending solution.

## Advantageous Effects

The microparticles containing moxidectin according to the present invention may exhibit a heartworm infection preventive effect over three months or more by releasing moxidectin sustainably over three months or more after administration, unlike conventional heartworm preventive drugs which need to be administered regularly every month due to their short half-life.
In addition, the microparticles produced according to the present invention have a smooth appearance, a uniform average particle diameter, and a narrow particle diameter distribution. Thus, the microparticles may prevent excessive release of moxidectin at an initial stage after administration of the microparticles, maintain the effective concentration of moxidectin at a constant level over 3 months or more by controlling the release of the drug, and reduce foreign body sensation and pain when applied by injection.

## Brief Description of Drawings

FIG. 1 shows experimental results for the moxidectin release patterns of microparticles according to Production Examples of the present invention.
FIG. 2 shows experimental results for the moxidectin release patterns of microparticles according to Production Examples of the present invention.
FIG. 3 shows experimental results for the moxidectin release patterns of microparticles according to Production Examples of the present invention.
FIG. 4 is an SEM photograph of microparticles according to one Production Example of the present invention.
FIG. 5 is an SEM photograph of microparticles according to one Production Example of the present invention.
FIG. 6 is an SEM photograph of microparticles according to one Production Example of the present invention.

FIG. 7 is an SEM photograph of microparticles according to one Production Example of the present invention.
FIG. 8 is an SEM photograph of microparticles according to one Production Example of the present invention.
FIG. 9 is an SEM photograph of microparticles according to one Production Example of the present invention.
FIG. 10 is an SEM photograph of microparticles according to one Production Example of the present invention.
FIG. 11 is an SEM photograph of microparticles according to one Production Example of the present invention.
FIG. 12 is an SEM photograph of microparticles according to one Production Example of the present invention.
FIG. 13 is an SEM photograph of microparticles according to one Production Example of the present invention.
FIG. 14 is an SEM photograph of microparticles according to one Production Example of the present invention.
FIG. 15 is an SEM photograph of microparticles according to one Production Example of the present invention.
FIG. 16 is an SEM photograph of microparticles according to one Production Example of the present invention.
FIG. 17 is an SEM photograph of microparticles according to one Production Example of the present invention.
FIG. 18 is an SEM photograph of microparticles according to one Production Example of the present invention.
FIG. 19 shows the results of measuring the plasma concentration of moxidectin after administering an injectable formulation containing microparticles according to one Example of the present invention to beagle dogs.

**Best Mode**

The present invention is directed to microparticles containing moxidectin and a biodegradable polymer, wherein the biodegradable polymer has an intrinsic viscosity of 0.1 dl/g to 1 dl/g, and the microparticles have an average diameter of 60 to 110 μm.

**Mode for Invention**

Hereinafter, embodiments of the present invention will be described in detail so that those of ordinary skill in the art to which the present invention pertains can easily carry out the present invention. However, the present invention may be embodied in a variety of different forms and is not limited to the embodiments described herein.

Microparticles containing moxidectin according to one embodiment of the present invention are microparticles containing moxidectin and a biodegradable polymer, wherein the biodegradable polymer has an intrinsic viscosity of 0.1 dl/g to 1 dl/g, and the microparticles have an average diameter of 60 to 110 μm.

As used herein, the term "moxidectin" refers to a compound represented by the following Formula 1, which is used as an animal heartworm preventive agent:

[Formula 1]

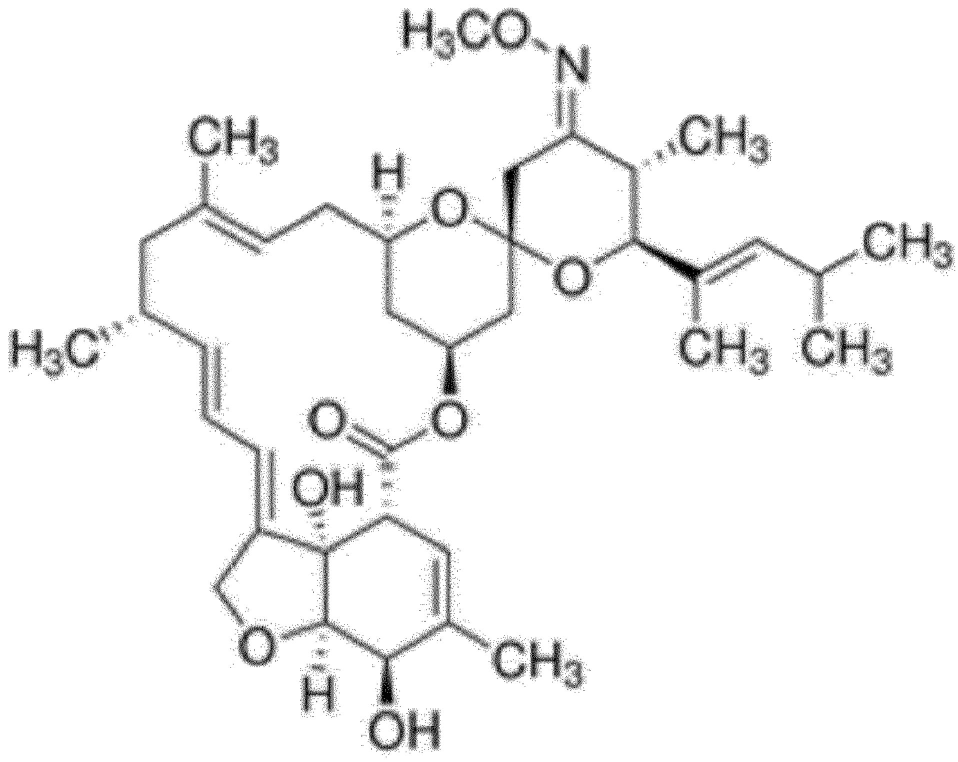

The microparticles are characterized by being spherical in shape and having a smooth surface.

In addition, the microparticles contain a biodegradable polymer and moxidectin, wherein the biodegradable polymer and moxidectin are uniformly mixed with each other, and moxidectin is distributed uniformly in the microparticles.

The microparticles may be used in the form of an injection, and the microparticles may be injected into an animal body. The microparticles injected into the animal body may release moxidectin slowly as the biodegradable polymer is degraded.

Regarding the release of moxidectin from the microparticles of the present invention, the biodegradable polymer on the surface is degraded, moxidectin starts to be released, and then, when a plurality of holes are formed inside the microparticles, moxidectin contained in the microparticles may be released into the body of the animal.

Due to the release of moxidectin as described above, the microparticles of the present invention may release moxidectin in the animal body over 3 months or more, and preferably may release moxidectin sustainably over 6 months or 12 months.

Moxidectin may be released at a concentration equal to or higher than a concentration suitable for the treatment or prevention of heartworm disease. This means that moxidectin is not simply sustainably released for a long period of time, but is sustainably released at a concentration equal to or higher than the plasma concentration at which the medicinal effect of moxidectin can be actually exerted.

The reason why the microparticles of the present invention may release moxidectin sustainably and exhibit the effect of preventing heartworm disease as described above is attributable to the above-described average diameter of the microparticles, the coefficient of variation (CV) to be described later, and the intrinsic viscosity of the biodegradable polymer.

The microparticles of the present invention may have an average diameter of 60 to 110 μm, 60 to 100 μm, or 70 to 100 μm. When the average diameter is within the above range, the microparticles may exhibit the effect of releasing moxidectin for a desired period, may be administered without foreign body sensation by injection, and may be prevented from being absorbed by macrophages *in vivo.*

The microparticles may have a coefficient of variation (CV) of 5% to 20%, 6% to 19%, 7% to 18.5%, or 8% to 18.5%. The coefficient of variation is a value indicating the monodispersity of particle size distribution, and a smaller CV value indicates a more uniform particle size.

The coefficient of variation may be calculated by the following Equation 2:

[Equation 2]

$$(\text{Standard deviation of diameter / average value of diameter})*100$$

As the microparticles having a coefficient of variation within the above range are included, it is possible to control the release pattern of moxidectin. In addition, as described later, when the microparticles are produced by the method of the present invention, microparticles having uniform size may be produced, and thus microparticles containing different biodegradable polymers may be produced and mixed together before use.

Microparticles containing a drug such as moxidectin may release the drug as the biodegradable polymer is degraded in the body as described above. Accordingly, the microparticles should have a uniform size so that they may release the drug sustainably for a desired period.

Specifically, if microparticles have a broad size distribution, a problem arises in that microparticles having a small particle size are rapidly degraded in the body or do not exhibit drug release due to macrophages. That is, even if microparticles with a small particle size release the drug, they act only at an initial stage in the body and cannot release the drug over a long period of time.

In addition, if the size of the microparticles is excessively large, the effect of releasing the drug at an initial stage may be insignificant, and the drug may be released over an excessively long period of time. The microparticles are intended to release the drug for a desired period, and if the drug is released for more than a desired period, it is not possible to control the release pattern of the drug, which may cause difficulty in repeated dosing.

Microparticles having various sizes include both small-sized microparticles and large-sized micro particles as described above. This means that the size of the microparticles cannot be controlled, which means that the release time of the drug cannot be controlled, which may cause difficulty in continuous use rather than one-time use.

The microparticles of the present invention may have an average particle diameter of 60 to 110 μm and a coefficient of variation of 5% to 20%. This means that the microparticles are distributed within the average diameter range, meaning that the microparticles comprise only microparticles having a uniform diameter.

As the microparticles having a uniform size as described above are included, when the microparticles are injected into the animal body, they may prevent excessive release of moxidectin at an initial stage and exhibit the effect of releasing moxidectin for 3 months, 6 months or 12 months.

The biodegradable polymer is selected from the group consisting of polylactide (PLA), polylactide-co-glycolide (PLGA), polyphosphazene, polyiminocarbonate, polyphosphoester, polyanhydride, polyorthoester, polycaprolactone, polyhydroxyvalerate, polyhydroxybutyrate, polyamino acids, and mixtures thereof.

Preferably, the biodegradable polymer may be selected from the group consisting of polylactide (PLA), polylactide-co-glycolide (PLGA), and mixtures thereof.

Specifically, the microparticles may contain polylactide (PLA), polylactide-co-glycolide (PLGA), or a mixture of polylactide (PLA) and polylactide-co-glycolide (PLGA).

More specifically, the microparticles may contain a combination of PLA and moxidectin, a combination of PLGA and moxidectin, or a combination of PLA, PLGA and moxidectin. When the microparticles contain both PLA and PLGA, both PLA and PLGA are used as the biodegradable polymer in the process of dissolving the biodegradable polymer and moxidectin in an organic solvent as described below, and thus the produced microparticles contain both PLA and PLGA.

In addition, the microparticles of the present invention may be contained in the sustained-release injectable composition as described below, wherein the microparticles contained may be selected from the group consisting of microparticles containing PLA and moxidectin, microparticles containing PLGA and moxidectin, microparticles containing PLA, PLGA and moxidectin, and mixtures thereof.

The microparticles contained in the injectable composition may contain only one type of biodegradable polymer, or contain two or more different types of biodegradable polymer, or comprise two or more different types of microparticles containing one or more types of biodegradable polymer. The injectable composition containing various types of microparticles as described above serves to control the release time of moxidectin.

The intrinsic viscosity of the biodegradable polymer may be 0.1 dl/g to 1 dl/g, 0.1 dl/g to 0.8 dl/g, 0.1 dl/g to 0.7 dl/g, or 0.1 dl/g to 0.6 dl/g. In addition, the molecular weight (MW) of the biodegradable polymer may be 10 kg/mol to 100 kg/mol, 11 kg/mol to 80 kg/mol, 12 kg/mol to 70 kg/mol, or 15 kg/mol to 65 kg/mol.

The microparticles of the present invention are intended to release moxidectin sustainably over 3 months, 6 months or 12 months, preferably for 6 months or 12 months, in the animal body.

Although the release of moxidectin from the microparticles is also associated with the average diameter of the particles as described above, it is also greatly influenced by the intrinsic viscosity. In general, the release of moxidectin may be delayed as the viscosity of the biodegradable polymer increases. However, this feature is not necessarily applied, and the intrinsic viscosity of the biodegradable polymer and the viscosity of moxidectin influence may each other, thereby affecting the degradation of the biodegradable polymer and the release of moxidectin.

The above-described biodegradable polymers have been approved by the FDA, etc. and may also be used for injection of human pharmaceutical drugs, and have different degradation periods specified depending on the type thereof. That is, specific polymers having various biodegradation periods, such as 2 to 3 months, 6 to 9 months, and 10 to 14 months, are used.
However, this means the biodegradation period of the biodegradable polymer, and when the biodegradable polymer is mixed with moxidectin to produce microparticles, moxidectin may affect the biodegradation period of the biodegradable polymer, thus changing the biodegradation period.
Therefore, in order to release moxidectin sustainably for a desired period in the body, a combination of a biodegradable polymer and moxidectin is important.
That is, for the purpose of releasing moxidectin for 3 months, the simple use of a biodegradable polymer having a biodegradation period of 3 months cannot achieve this purpose, and it is necessary to examine whether 3-month biodegradation is possible by a combination of moxidectin and a biodegradable polymer.
Therefore, in the present invention, the intrinsic viscosity of the biodegradable polymer is limited to the range of 0.1 dl/g to 1 dl/g. When a biodegradable polymer having an intrinsic viscosity within the above range is used in combination with moxidectin, it may exhibit the effect of releasing moxidectin sustainably for a desired period of time.
The microparticles may contain the biodegradable polymer and moxidectin at a weight ratio of 2:1 to 12:1, 4:1 to 10:1, or 9:1. When the microparticles contain the biodegradable polymer and moxidectin at a weight ratio within the above range, they may prevent excessive release of moxidectin at an initial stage after injection into the animal body and exhibit the effect of sustainably releasing moxidectin for a desired period of time.
A general injectable formulation may cause side effects due to excessive release of moxidectin at an initial stage, but the microparticles according to the present invention may prevent excessive release of moxidectin at an initial stage even after administration by injection and exhibit the effect of sustainably releasing moxidectin for a desired period of time.
In addition, sustained release of moxidectin for 3 months, 6 months, or 12 months as described above means that moxidectin is released at a level capable of exhibiting the effect of preventing or treating heartworm disease.
In addition, the microparticles may have a value of 0.3 to 3 as calculated by the following Equation 1, which represents the plasma concentration of moxidectin measured after mixing the microparticles containing moxidectin with a suspending solution to obtain an injectable formulation and administering the injectable formulation to a plurality of beagle dogs:

[Equation 1]

$$C_{max\text{-}peak\ n}/C_{max\text{—}peak\ n+1}$$

wherein

$C_{max\text{-}peak\ n}$ is an $n^{th}$ Cmax value after administration of the injectable formulation, and
$C_{max\text{-}peak\ n+1}$ is an $n+1^{st}$ Cmax value measured when the plasma concentration of moxidectin increases again after the $n^{th}$ Cmax value.

Equation 1 above indicates a value obtained by measuring the plasma concentration of moxidectin after administering the microparticles of the present invention to beagle dogs by injection. The value calculated according to Equation 1 may be 0.3 to 3, 0.5 to 3, or 0.5 to 2.5. When the ratio of the peak values of the plasma concentration of moxidectin is within the above range, it is possible to achieve the long-term release of moxidectin for a desired period of time.
Equation 1 above indicates the relationship between the plasma concentration peaks of moxidectin shown by the injectable formulation containing microparticles containing different types of biodegradable polymers.
Specifically, as described above, the microparticles of the present invention may be produced using two or more different types of biodegradable polymers. The plurality of microparticles may contain only one type of biodegradable polymer, but they may also contain different biodegradable polymers. That is, the microparticles may include microparticles containing PLGA and moxidectin and microparticles containing PLA and moxidectin.
As shown in Equation 1 above, the microparticles containing different biodegradable polymers may show an $n^{th}$ maximum plasma concentration (Cmax-peak n) of moxidectin, and then an $n+1^{st}$ maximum plasma concentration (Cmax-peak n+1) of moxidectin.
The $n^{th}$ maximum plasma concentration of moxidectin means the maximum plasma concentration of moxidectin

after injection, which is the maximum value in the zone in which the plasma concentration value of moxidectin increases gradually and then tends to decrease again, and the n+1$^{st}$ maximum plasma concentration of moxidectin means the maximum value in the zone in which the plasma concentration value of moxidectin tends to increase again after the n$^{th}$ maximum plasma concentration of moxidectin and then tends to degrease again. Thus, the injectable formulation containing the microparticles of the present invention may show a plurality of maximum plasma concentration values. In the n$^{th}$ maximum plasma concentration (Cmax-peak n) of moxidectin and the n+1$^{st}$ maximum plasma concentration (Cmax-peak n+1) of moxidectin, the n$^{th}$ maximum plasma concentration value of moxidectin may be greater than the n+1$^{st}$ maximum plasma concentration value of moxidectin, or the n+1$^{st}$ maximum plasma concentration value of moxidectin may be greater than the n$^{th}$ maximum plasma concentration value of moxidectin.

The plasma concentration peak trend as described above is changed as the release pattern of moxidectin changes depending on the type of biodegradable polymer. The microparticles of the present invention are produced by the production method to be described later, and the microparticles produced by the production method of the present invention may be precisely controlled to have a uniform particle size. For this reason, different types of microparticles may be produced using different biodegradable polymers and mixed together before use.

The microparticles produced using different biodegradable polymers as described above may exhibit the respective characteristic moxidectin release patterns, and a plurality of plasma concentration peaks may appear depending on the characteristic release pattern.

When the microparticles are produced using different biodegradable polymers as described above, the biodegradable polymers have different biodegradation rates. Based on this characteristic, microparticles capable of exhibiting a moxidectin release effect at an initial stage after administration by injection and microparticles capable of exhibiting a moxidectin release effect at a late stage may be mixed together before use, and thus may exhibit the effect of releasing moxidectin sustainably over a long period of time.

Meanwhile, the microparticles of the present invention are characterized in that the maximum plasma concentration (Cmax) of moxidectin appears after a specific time point after administration into the body.

After administration of the microparticles of the present invention, the maximum plasma concentration (Cmax) of moxidectin in the body may be affected by the average diameter of the microparticles, the size distribution of the particles, the type of biodegradable polymer, and the like.

For example, the microparticles of the 3- to 6-month formulation may exhibit a maximum plasma concentration (Cmax) within 10 to 90 days after administered into the body. Specifically, the microparticles of the present invention may inhibit excessive release of moxidectin at an initial stage, and after injection into the body, the release amount of moxidectin may increase slowly, the maximum plasma concentration may appear within the range of 10 to 90 days, and then the release amount of moxidectin may decrease, and moxidectin may be released sustainably for 3 months or 6 months. Contrary to the above, when only one type of biodegradable polymer is used, a plurality of maximum plasma concentration peaks of moxidectin does not appear, and only a single maximum plasma concentration peak appears, and the maximum plasma concentration may appear within the range of 10 to 90 days after injection.

In addition, the 12-month formulation may exhibit the maximum plasma concentration (Cmax) between 10 days and 90 days, like the above-described 3-month formulation or 6-month formulation. However, as described above, the maximum plasma concentration (Cmax) may be affected by the average diameter of the microparticles, the size distribution of the particles, the type of biodegradable polymer, and the like.

The range of days showing the maximum plasma concentration may vary depending on the administration subject and administration dose, but in the embodiment of the present invention, when the total dose of moxidectin administered to beagle dogs is 0.2 mg/kg to 1 mg/kg, the maximum plasma concentration (Cmax) of moxidectin may appear within 10 to 90 days. When the maximum blood concentration (Cmax) of moxidectin appears within the above range, sustained release of moxidectin for 3 months, 6 months, or 12 months is possible, and preferably, moxidectin may be sustainably for 6 months or 12 months.

The microparticles containing moxidectin were mixed with a suspending solution to obtain an injectable formulation, and the injectable formulation was administered to a plurality of beagle dogs. The injectable formulation was administered so as to satisfy the value calculated by Equation 1 above. The same injectable formulation was administered to 10 beagle dogs at a moxidectin dose of 0.2 mg/kg, and the plasma moxidectin concentrations of the beagle dogs were measured. The standard deviation of the plasma concentration of moxidectin between the beagle dogs at the same time point could be 0.01 to 10, 0.01 to 5, or 0.01 to 3.

The above-described standard deviation value of the plasma concentration of moxidectin means that the produced microparticles have excellent uniformity, and excellent reproducibility in *in vivo* test results means that the efficacy and safety of moxidectin after administration are excellent.

Even when the same injectable formulation is administered to beagle dogs, a difference in the plasma concentration of moxidectin between the beagle dogs may occur. This is due to the difference in drug-metabolizing enzymes between the beagle dogs, and even when the same injectable formulation is administered, the metabolic rate of the drug released *in vivo* may differ between the beagle dogs.

However, in order to provide a sustained-release formulation, it is necessary to control the plasma concentration value of moxidectin so as not to significantly change after administration into the body. This plasma concentration value may be a factor that is affected by the quality of the produced particles containing moxidectin.

The microparticles of the present invention are produced by a production method to be described later, and may be produced to have a very uniform particle size, a smooth particle surface and a perfectly spherical shape.

That is, as the microparticles having a uniform size and smooth surface appearance as described above are produced, even when these microparticles are administered to beagle dogs by injection, a difference in the plasma concentration of moxidectin may occur due to a difference between the beagle dogs, but this difference is insignificant.

On the other hand, unlike the present invention, in the case of microparticles having a non-uniform particle size or a non-smooth surface appearance, the microparticles contained in the injectable formulation have various particle sizes and different surface appearances, and thus when these microparticles are administered to beagle dogs or the like, the difference in the measured plasma concentration values of moxidectin is significant not only due to the difference between the beagle dogs but also due to the difference between the microparticles.

The sustained-release injectable composition containing moxidectin according to another embodiment of the present invention may contain: the microparticles containing moxidectin; and a suspending solution.

The suspending solution contains an isotonic agent, a suspending agent, and a solvent.

More specifically, the isotonic agent may be selected from the group consisting of D-mannitol, maltitol, sorbitol, lactitol, xylitol, sodium chloride, and mixtures thereof, with D-mannitol being preferred, but the isotonic agent is not limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, with sodium carboxymethylcellulose and polysorbate 80 being preferred, but the suspending agent is not limited to the above examples.

As the solvent, water for injection may be used, and any solvent that may be used as water for injection may be used without limitation.

A method for producing microparticles containing moxidectin according to still another embodiment of the present invention may comprise steps of: 1) preparing an oil-phase solution by dissolving a biodegradable polymer and moxidectin in an organic solvent; 2) preparing a water-phase solution by dissolving a surfactant in water; 3) injecting the oil-phase solution of step 1) into a microchannel in a straight-line direction and allowing the oil-phase solution to flow; 4) injecting the water-phase solution of step 2) into a microchannel formed on both side surfaces or one side surface so as to form a junction with the microchannel through which the oil-phase solution of step 3) flows in a straight-line direction, allowing the water-phase solution to flow, and allowing the flow of the oil-phase solution to intersect with the flow of the water-phase solution, thereby producing microparticles containing moxidectin distributed uniformly therein; 5) collecting the microparticles produced at the junction in step 4); 6) stirring the microparticles collected in step 5) to evaporate and remove the organic solvent from the microparticles; and 7) washing and drying the microparticles resulting from step 6), wherein the biodegradable polymer has an intrinsic viscosity of 0.1 dl/g to 1 dl/g, and the microparticles have an average diameter of 60 to 110 $\mu$m.

Step 1) is a step of preparing an oil-phase solution by dissolving moxidectin and a biodegradable polymer in an organic solvent, wherein the biodegradable polymer is polylactide (PLA), preferably polylactide-co-glycolide (PLGA) or polylactide (PLA), without being limited thereto.

In addition, the organic solvent is water-immiscible, and may be, for example, any one or more selected from the group consisting of chloroform, chloroethane, dichloroethane, dichloromethane, trichloroethane, and mixtures thereof, with dichloromethane being preferred, without being limited thereto. In addition to the above-listed organic solvents, any organic solvent may be used without limitation, as long as it is capable of dissolving the biodegradable polymer and moxidectin and may be easily selected by those skilled in the art.

Step 1) is a step of preparing an oil-phase solution by dissolving moxidectin and a biodegradable polymer, and as the solvent, the organic solvent described above is used. The organic solvent is used to completely dissolve moxidectin and the biodegradable polymer based on the dissolution properties thereof. More specifically, the oil-phase solution is prepared by dissolving moxidectin and the biodegradable polymer in the organic solvent.

The weight ratio between the biodegradable polymer and moxidectin in the oil-phase solution may be 2:1 to 12:1, 4:1 to 10:1, or 9:1. When the weight ratio is within the above range, moxidectin may be released sustainably for a long period of time by degradation of the biodegradable polymer.

If the weight ratio between moxidectin and the biodegradable polymer is less than 1:2, that is, if the content of the biodegradable polymer is lower than the lower limit of the above weight ratio, a problem may arise in that, because the content of the biodegradable polymer is lower than the content of moxidectin, it is difficult to produce microparticles in which moxidectin is uniformly distributed in spherical biodegradable polymer particles. If the weight ratio between moxidectin and the biodegradable polymer is more than 1:12, that is, if the content of the biodegradable polymer is higher than the upper

limit of the above weight ratio, a problem may arise in that, because the content of moxidectin in the sustained-release particles is low, a large amount of sustained-release particles need to be administered in order to administer the drug at a desired concentration.

More specifically, the content of the biodegradable polymer in the oil-phase solution is 10 to 20 wt%, preferably 15 wt%, without being limited thereto.

Step 2) is a step of preparing a water-phase solution by dissolving a surfactant in water. As the surfactant, any surfactant may be without limitation as long as it may help the biodegradable polymer solution to form a stable emulsion. Specifically, the surfactant may be any one or more selected from the group consisting of nonionic surfactants, anionic surfactants, cationic surfactants, and mixtures thereof, and more specifically, may be any one or more selected from the group consisting of methylcellulose, polyvinylpyrrolidone, lecithin, gelatin, polyvinyl alcohol, polyoxyethylene sorbitan fatty acid ester, polyoxyethylene castor oil derivatives, sodium lauryl sulfate, sodium stearate, ester amine, linear diamine, fatty amines, and mixtures thereof, with polyvinyl alcohol being preferred, but the surfactant is not limited to the above examples.

The content of the surfactant in the water-phase solution may be 0.1 to 1.0 wt%, 0.2 to 0.5 wt%, or 0.25 wt%. The rest is water.

Step 3) is a step of injecting the oil-phase solution and the water-phase solution into microchannels formed on a wafer or a glass substrate and allowing the oil-phase solution and the water-phase solution to flow.

Conventionally, microchannels used to prepare the same microparticles as used in the present invention are fabricated by forming seven microchannels on a silicon wafer. The microchannels may include a channel through which a water-phase solution flows, a channel through which an oil-phase solution flows, and a channel through which the emulsion produced after formation of an intersection between the water-phase solution and the oil-phase solution moves.

However, in the case of a chip in which a total of seven channels are formed on the conventional silicon wafer, a small number of microchannels are formed therein. Thus, when the seven microchannels are used to produce microparticles simultaneously, it is possible to produce microparticles having a uniform size by controlling the pressures of the oil-phase solution and the water-phase solution.

However, as the number of microchannels formed in the chip increases, a problem arises in that it is not easy to produce microparticles having a uniform size by controlling the pressures of the oil-phase solution and the water-phase solution.

That is, if 10 or more microchannels are formed on one plane, problems arise in that it is not easy to supply the water-phase solution and the oil-phase solution to Nos. 1 to 10 channels at the same pressure, and in that the size and particle size distribution of the produced microparticles change due to the non-uniformity of pressure distribution in each microchannel.

Accordingly, the present invention is characterized in that the flow rate ratio between the oil-phase solution and the water-phase solution that flow into the channels is maintained at 1:45 or more so that microparticles having a uniform size may be produced even in a chip having 10 or more microchannels formed therein.

Specifically, when the flow rate of the oil-phase solution is adjusted to 110 μl/min and the flow rate of the water-phase solution is adjusted to a flow rate of 5,000 μl/min, it is possible to produce a uniform emulsion at the junction.

The production of microparticles using microchannels as described above is performed using microfluidics. The microfluidics is a technique of producing microparticles having a uniform particle size distribution using microfluidic engineering technology based on the difference in polarity between the oil-phase solution and the water-phase solution, and produces an emulsion through the repulsive force between the fluids when passing through the microchannels.

When a conventional chip having a single channel or about 10 channels is used, it is possible to control the flow rates by adjusting the ratio of the pressures of the oil-phase solution and the water-phase solution which are injected into the channels. However, when a chip having multiple channels integrated thereon is used, it is important to control the ratio of the flow rates of the oil-phase solution and the water-phase solution, not the pressures of the oil-phase solution and the water-phase solution that are injected into the channels.

Whether it is possible to produce microparticles using microchannels depends on the ratio of the flow rates of the oil-phase solution and the water-phase solution. When the fluids flowing in the microchannels form a laminar flow and are constantly cut off by the repulsive force between the fluids at the junction between the channels, they drip, but when the ratio of the flow rates of the oil-phase solution and the water-phase solutions is out of the range specified in the present invention, jetting, not dripping, occurs, and thus a uniform emulsion is not produced.

FIG. 1 is a photograph of an emulsion formed when the water-phase solution and the oil-phase solution have a flow rate ratio within the range specified in the present invention and drip at the junction between channels.

It can be seen that the particles in the emulsion shown in FIG. 1 have a uniform particle size distribution of 170 to 190 μm, a perfectly spherical shape, and a smooth surface shape.

Meanwhile, FIG. 2 is a conceptual diagram of an emulsion formed by dripping at the junction between channels and an emulsion which may be formed by jetting at the junction.

It can be confirmed that, in the case of dripping as described above, an emulsion containing particles having a

uniform diameter as shown in FIG. 1 is formed, but in the case of jetting, an emulsion containing particles having different diameters is formed.

The ratio of the flow rates of the oil-phase solution and the water-phase solution may be 1:45 or more, and may be 1:45 to 1:125. If the ratio is less than 1:45, a problem may arise in that jetting occurs, and thus the particles do not have a uniform size distribution corresponding to a CV of 10% or less, and if the ratio is more than 1:125, the fluids completely drip and the produced particles have a suitable size distribution, but a problem may arise in that the production quantity decreases because the amount of water-phase solution used increases as the flow rate of the water-phase solution increases.

More specifically, the microchannels may be formed on a material selected from the group consisting of a glass substrate, a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited thereto.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, the silicon wafer is etched by deep ion reactive etching (DRIE) using the aluminum as a mask, the aluminum is removed, glass is anodically bonded onto the wafer and hermetically sealed, thereby fabricating the microchannels.

The microchannel has an average diameter of 160 to 200 μm, preferably 180 μm, without being limited thereto. If the average diameter of the microchannel is 160 μm or less, small-sized microparticles with a diameter of less than 50 μm may be produced, which may affect the release and *in vivo* absorption of the effective drug. In addition, if the average diameter of the microchannel is 200 μm or more, the produced microparticles have an average size of more than 120 μm, foreign body sensation and pain may increase when the particles are administered by injection, and the particle size distribution of the produced particles may increase as the diameter of the microchannel increases, making it difficult to produce microparticles having a uniform particle size distribution.

In addition, the average diameter of the microchannel is closely related not only to the average diameter of the particles, but also to the ratio of the flow rates (μl/min) of the oil-phase solution and the water-phase solution.

In addition, the cross-sectional width (w) and cross-sectional height (d) of the microchannel are closely related to the average diameter (d') of the produced microparticles. The cross-sectional width (w) of the microchannel is in the ratio range of 0.7 to 1.3 with respect to the average diameter (d') of the microparticles, and the cross-sectional height (d) of the microchannel is in the ratio range of 0.7 to 1.3 with respect to the average diameter (d') of the microparticles.

That is, when the average diameter (d') of the microparticles to be produced is determined, it is possible to produce microparticles having a desired size only when the cross-sectional width (w) and height (d) of the microchannel are set to the ratio range of 0.7 to 1.3 with respect to d'.

Step 3) is a step of injecting the oil-phase solution and the water-phase solution into a first microchannel and a second microchannel, respectively, which have a junction formed therebetween, and allowing the oil-phase solution and the water-phase solution to flow under the above-described flow rate conditions.

That is, the oil-phase solution flows along the first microchannel, and the water-phase solution flows along the second microchannel configured to form a junction with the first microchannel, and meets the flow of the oil-phase solution.

More specifically, when the oil-phase solution is injected into the first microchannel and the water-phase solution is injected into the second microchannel, the flow rate ratio between the oil-phase solution and the water-phase solution may be 1:45 or more, or 1:45 to 1:125.

As described above, in the production of microparticles according to the conventional art, it is possible to produce microparticles having a certain diameter by controlling pressure, but in the case of a chip in which 10 or more microchannels are integrated, it is impossible to produce microparticles having a uniform diameter by controlling pressure. In this case, it is possible to produce microparticles having a uniform diameter by controlling the flow rate ratio between the oil-phase solution and the water-phase solution.

As described above, when the flow rates of the oil-phase solution and the water-phase solution are made different from each other, the water-phase solution having a higher flow rate compresses the oil-phase solution at the point where the flow of the oil-phase solution and the flow of the water-phase solution meet each other. At this time, the dripping phenomenon appears due to the repulsive force between the oil-phase solution and the water-phase solution, thereby producing spherical microparticles in which the biodegradable polymer and moxidectin are uniformly distributed. More specifically, the microparticles formed are microparticles in which moxidectin is uniformly distributed in the spherical biodegradable polymer.

Step 5) is a step of collecting the microparticles. In this step, the microparticles are collected in a bath containing the water-phase solution to prevent aggregation of the initially produced microparticles.

Step 5) is performed using the water-phase solution prepared in step 2), that is, a mixed solution of the surfactant

and water. Specifically, a portion of the water-phase solution prepared in step 2) is injected into the microchannel, and the other portion is transferred into the bath in step 5) and used to prevent aggregation of the collected microparticles.

Step 6) is a step of removing an organic solvent from the microparticles collected in the bath. In this step, an organic solvent present inside the microparticles is evaporated and removed by stirring the microparticles at a predetermined stirring speed at a predetermined temperature. Specifically, step 6) may include steps of: 6-1) subjecting the microparticles to first stirring at a speed of 150 to 350 rpm at 15 to 20°C for 50 to 70 minutes; 6-2) subjecting the microparticles to second stirring at a speed of 250 to 450 rpm at 20 to 30°C for 50 to 70 minutes; and 6-3) subjecting the microparticles to third stirring at a speed of 450 to 650 rpm at 40 to 60°C for 3 to 9 hours.

The first and second stirring steps are performed at different stirring speeds at different temperatures for different stirring times.

As described above, step 6) is characterized in that the stirring temperature is higher in the second stirring step than in the first stirring step. As the stirring temperature is increased stepwise, it is possible to control the evaporation rate of the organic solvent present inside the microparticles. That is, it is possible to slowly evaporate the organic solvent present inside the microparticles, thereby producing microparticles.

The temperature at which the oil-phase solution and the water-phase solution flow through the microchannels is also 15 to 20°C, preferably 17°C. That is, after the solutions flow through the microchannels and microparticles are produced at a junction therebetween, the collected microparticles are constantly maintained at a low temperature of 15 to 20°C until they are stirred in the first stirring step. It is possible to produce and maintain spherical particles only when microparticles are maintained at low temperature during the production thereof. That is, if a low-temperature condition is not used, a problem arises in that it is difficult to produce particles having a uniform spherical shape.

Thereafter, in the second stirring step, the temperature is increased gradually and the stirring time is increased so that the organic solvent present inside the microparticles may move slowly to the surface and evaporate from the surface, thereby minimizing the effect of the organic solvent on the surface appearance of the microparticles. That is, if the organic solvent is rapidly evaporated, a problem may arise in that the surfaces of the microparticles are not smooth and pores are formed, due to evaporation of the organic solvent. In order to prevent this problem, the evaporation rate of the organic solvent may be controlled by increasing the temperature gradually as described above and also increasing the stirring process time, and due to this control of the evaporation rate of the organic solvent, it is possible to control the surface appearance of the produced microparticles.

In the third stirring step, after the organic solvent in the emulsion is extracted with the external water phase, the external water phase is heated to a temperature near the boiling point of the organic solvent and stirred to remove the saturated organic solvent from the water phase, thereby facilitating the removal of the organic solvent remaining in the microparticles.

Lastly, step 7) is a step of washing and drying the microparticles. In this step, the microparticles from which the organic solvent on the surface has been completely removed by stirring are washed several times with sterile filtered purified water to remove the surfactant remaining on the microparticles, and then the microparticles are freeze-dried.

The microparticles finally produced are in a form in which moxidectin is uniformly distributed in microparticles composed of the spherical biodegradable polymer, and contain the biodegradable polymer and moxidectin at a weight ratio of 2:1 to 12:1.

The weight ratio between the moxidectin and biodegradable polymer contained in the microparticles is the same as the weight ratio in the oil-phase solution. Specifically, as the oil-phase emulsion particles are produced by passage through the microchannels and the organic solvent is completely removed from the emulsion, the produced microparticles may contain moxidectin and the biodegradable polymer at the same weight ratio as the weight ratio in the oil-phase solution.

Production Example 1

Production of microparticles containing moxidectin

An oil-phase solution was prepared by dissolving polylactide-co-glycolide (PLGA) (having a viscosity of 0.2 dl/g, a molecular weight (MW) of 17 kg/mol, and a ratio of lactide to glycolide of 75:25 and containing a carboxyl group at the end) and moxidectin in dichloromethane. Here, the content of polylactide-co-glycolide in the oil-phase solution was 15 wt%, and the weight ratio between polylactide-co-glycolide and moxidectin was 9:1.

A water-phase solution containing 0.25 wt% of polyvinyl alcohol was prepared by mixing polyvinyl alcohol as a surfactant with water.

The oil-phase solution and the water-phase solution were injected into microchannels formed on a silicon wafer and allowed to flow. The oil-phase solution and water-phase solution injected into the respective microchannels were maintained at a flow rate ratio of 1:50 and at a temperature of 17°C.

Microparticles produced at the intersection between the flow of the oil-phase solution and the flow of the water-phase solution were collected in a water tank containing the water-phase solution. The microparticles collected in the bath were subjected to first stirring at a speed of 250 rpm for 1 hour at 17°C, and then subjected to second stirring at a speed of 350 rpm for 1 hour at 25°C, and then subjected to third stirring at a speed of 550 rpm for 4 hours at an increased temperature of 55°C.

After completion of stirring, the microparticles were washed several times with sterile filtered purified water and freeze-dried, thereby producing microparticles.

Production Example 2-1

Microparticles were produced in the same manner as in Production Example 1, except that polylactic acid having a viscosity of 0.2 dl/g and a molecular weight (MW) of 17 kg/mol and containing a carboxyl group at the end was used instead of polylactide-co-glycolide.

Production Example 2-2

Microparticles were produced in the same manner as in Production Example 1, except that polylactic acid having a viscosity of 0.2 dl/g and a molecular weight (MW) of 17 kg/mol and containing a carboxyl group at the end was used instead of polylactide-co-glycolide and that the oil-phase solution contained 10 wt% of polylactic acid.

Production Example 3-1

Microparticles were produced in the same manner as in Production Example 1, except that polylactic acid having a viscosity of 0.4 dl/g and a molecular weight (MW) of 45 kg/mol and containing a carboxyl group at the end was used instead of polylactide-co-glycolide.

Production Example 3-2

Microparticles were produced in the same manner as in Production Example 1, except that polylactic acid having a viscosity of 0.4 dl/g and a molecular weight (MW) of 45 kg/mol and containing a carboxyl group at the end was used instead of polylactide-co-glycolide and that the oil-phase solution contained 10 wt% of polylactic acid.

Production Example 4

Microparticles were produced in the same manner as in Production Example 1, except that polylactic acid having a viscosity of 0.4 dl/g and a molecular weight (MW) of 45 kg/mol and containing an ester group at the end was used instead of polylactide-co-glycolide and that the oil-phase solution contained 10 wt% of polylactic acid.

Production Example 5

Microparticles were produced in the same manner as in Production Example 1, except that polylactic acid having a viscosity of 0.5 dl/g and a molecular weight (MW) of 61 kg/mol and containing an ester group at the end was used instead of polylactide-co-glycolide and that the oil-phase solution contained 10 wt% of polylactic acid.

Production Example 6

Microparticles were produced in the same manner as in Production Example 1, except that polylactide-co-glycolide (PLGA) having a viscosity of 0.2 dl/g, a molecular weight (MW) of 17 kg/mol, and a ratio of lactide to glycolide of 75:25 and containing a carboxyl group at the end, and a polylactide (PLA) having a viscosity of 0.2 dl/g and a molecular weight (MW) of 17 kg/mol and containing a carboxyl group at the end were used as biodegradable polymers at a weight ratio of 1:1, and that the oil-phase solution contained 10 wt% of polylactide-co-glycolide and polylactic acid.

Production Example 7-1

Microparticles were produced in the same manner as in Production Example 1, except that a polylactide (PLA) having a viscosity of 0.2 dl/g and a molecular weight (MW) of 17 kg/mol and containing a carboxyl group at the end, and a polylactide (PLA) having a viscosity of 0.4 dl/g and a molecular weight (MW) of 45 kg/mol and containing a carboxyl group at the end were used as biodegradable polymers at a weight ratio of 1:1.

Production Example 7-2

Microparticles were produced in the same manner as in Production Example 1, except that a polylactide (PLA) having a viscosity of 0.2 dl/g and a molecular weight (MW) of 17 kg/mol and containing a carboxyl group at the end, and a polylactide (PLA) having a viscosity of 0.4 dl/g and a molecular weight (MW) of 45 kg/mol and containing a carboxyl group at the end were used as biodegradable polymers at a weight ratio of 1:1, and that the total content of the polylactic acids in the oil-phase solution was 10 wt%.

Production Example 8

Microparticles were produced in the same manner as in Production Example 1, except that a polylactide (PLA) having a viscosity of 0.2 dl/g and a molecular weight (MW) of 17 kg/mol and containing a carboxyl group at the end, and a polylactide (PLA) having a viscosity of 0.4 dl/g and a molecular weight (MW) of 45 kg/mol and containing an ester group at the end were used as biodegradable polymers at a weight ratio of 1:1, and that the total content of the polylactic acids in the oil-phase solution was 10 wt%.

Production Example 9-1

Microparticles were produced in the same manner as in Production Example 1, except that a polylactide (PLA) having a viscosity of 0.2 dl/g and a molecular weight (MW) of 17 kg/mol and containing a carboxyl group at the end, and a polylactide (PLA) having a viscosity of 0.4 dl/g and a molecular weight (MW) of 45 kg/mol and containing a carboxyl group at the end were used as biodegradable polymers at a weight ratio of 1:3.

Production Example 9-2

Microparticles were produced in the same manner as in Production Example 1, except that a polylactide (PLA) having a viscosity of 0.2 dl/g and a molecular weight (MW) of 17 kg/mol and containing a carboxyl group at the end, and a polylactide (PLA) having a viscosity of 0.4 dl/g and a molecular weight (MW) of 45 kg/mol and containing a carboxyl group at the end were used as biodegradable polymers at a weight ratio of 1:3, and that the total content of the polylactic acids in the oil-phase solution was 10 wt%.

Production Example 10

Microparticles were produced in the same manner as in Production Example 1, except that a polylactide (PLA) having a viscosity of 0.4 dl/g and a molecular weight (MW) of 45 kg/mol and containing an ester group at the end, and a polylactide (PLA) having a viscosity of 0.5 dl/g and a molecular weight (MW) of 61 kg/mol and containing an ester group at the end were used as biodegradable polymers at a weight ratio of 1:1, and that the total content of the polylactic acids in the oil-phase solution was 10 wt%.

Production Example 11

Microparticles were produced in the same manner as in Production Example 1, except that a polylactide-co-glycolide (PLGA) having a viscosity of 0.2 dl/g, a molecular weight (MW) of 17 kg/mol, and a ratio of lactide to glycolide of 50:50 and containing a carboxyl group at the end, a polylactide-co-glycolide (PLGA) having a viscosity of 0.2 dl/g, a molecular weight (MW) of 17 kg/mol, and a ratio of lactide to glycolide of 75:25 and containing a carboxyl group at the end, and a polylactide (PLA) having a viscosity of 0.2 dl/g and a molecular weight (MW) of 17 kg/mol and containing a carboxyl group at the end were used as biodegradable polymers at a weight ratio of 1: 1: 4.

Example 1

The microparticles of Production Example 1 and the microparticles of Production Example 2-1 were mixed together at a weight ratio of 1:2, and 2.0 mL (based on one vial) of a suspending solvent was added thereto, and then the microparticle mixture was uniformly suspended in the suspending solvent, thereby preparing a composition for subcutaneous injection.

The suspending solvent had the composition shown in Table 1 below.

[Table 1]

| Volume | Purpose of mixing | Component name | Quantity | Unit |
|---|---|---|---|---|
| 2.0 mL | Isotonic agent | D-mannitol | 100.0 | mg |
| | Suspending agent | Sodium carboxymethylcellulose | 10.0 | mg |
| | Suspending agent | Polysorbate 80 | 10.0 | mg |
| | Solvent | Water for injection | Remainder | |

Example 2

A composition for subcutaneous injection was prepared in the same manner as in Example 1, except that the microparticles of Production Example 1 and the microparticles of Production Example 2-1 were mixed together at a weight ratio of 1:1 and used.

Example 3

The experiment was conducted in the same manner as in Example 1, except that 50% of the dose administered in Example 1 was used. The microparticles of Production Example 1 and the microparticles of Production Example 2-1 were mixed together at a weight ratio of 1:2 in the same manner as in Example 1 and administered.

Experimental Example 1

Release pattern

In order to examine the release of moxidectin from the microparticles of Preparation Examples 1, 2-1, 4, 5, a release test was performed.

The release test solution used in the release test was a solution containing 2% Tween 20 in purified water. 100 mg of the microparticles were placed in an injection bottle containing 100 ml of the release test solution, sealed, and then shaken at 120 rpm at 55°C, and the difference in dissolution rate was checked.

The test results are shown in FIG. 1.

The microparticles of Production Example 1 showed 50% release of moxidectin at 15 hours and are applicable to a 3-month formulation, and the microparticles of Production Example 2-1 showed 50% release of moxidectin at 20 hours and are applicable to a 6-month formulation.

The microparticles of Production Example 4 showed 50% release of moxidectin at 84 hours and are applicable to a formulation that releases moxidectin for a longer period of time than Production Example 1 and Production Example 2-1.

In addition, it can be confirmed that the microparticles of Production Example 5 take a longer time to release 50% of moxidectin than the microparticles of Production Example 3.

Experimental Example 2

Release pattern

In order to examine the release of moxidectin from the microparticles of Preparation Examples 2-1, 3-1, 9-1, 7-1 and 11, a release test was performed.

The release test solution used in the release test was a solution containing 2% Tween 20 and 0.001 M sodium bicarbonate in purified water. 100 mg of the microparticles were placed in an injection bottle containing 100 ml of the release test solution, sealed, and then shaken at 120 rpm at 55°C, and the difference in dissolution rate was checked.

The test results are shown in FIG. 2.

In the release test for the microparticles having an average diameter (MV) of about 95 μm and similar sizes, the release pattern did differ depending on the molecular weight of the polymer constituting the microparticles. When comparing the moxidectin release rate between the microparticles of Production Examples 2-1, 3-1, 7-1 and 9-1, it was confirmed that the microparticles released moxidectin for a longer time as the content of the polylactide having a molecular weight of (MW) of 45 kg/mol rather than the polylactide having a molecular weight (MW) of 17 kg/mol increased.

It was confirmed that the release delay time was different between Production Examples 7-1 in which the ratio of the polylactide having a molecular weight (MW) of 17 kg/mol to the polylactide having a molecular weight (MW) of 45 kg/mol was 1:1 and Production Example 9-1 in which the ratio was 1:3.

In addition, it can be confirmed that the microparticles of Production Example 11, which contain polylactide-co-glycolide (PLGA), released moxidectin faster than those of the other Production Examples.

Experimental Example 3

Release pattern

In order to examine the release of moxidectin from the microparticles of Preparation Examples 2-2, 3-2, 4, 7-2, 8 and 9-2, a release test was performed.
The release test solution used in the release test was a solution containing 2% Tween 20 and 0.001 M sodium bicarbonate in purified water. 100 mg of the microparticles were placed in an injection bottle containing 100 ml of the release test solution, sealed, and then shaken at 120 rpm at 55°C, and the difference in dissolution rate was checked.
The test results are shown in FIG. 3.
In the release test for the microparticles having an average diameter (MV) of about 85 μm and similar sizes, the release pattern did differ depending on the molecular weight and end group of the polymer constituting the microparticles. When comparing the moxidectin release rate between the microparticles of the Production Examples, it was confirmed that the microparticles released moxidectin for a longer time as the content of the polylactide having a molecular weight of (MW) of 45 kg/mol rather than the polylactide having a molecular weight (MW) of 17 kg/mol increased.
In addition, when comparing the microparticles of Production Examples 3-2 and 4, it can be confirmed that the release of moxidectin was more delayed when the biodegradable polymer contained an ester group as the end group than when the biodegradable polymer contained a carboxyl group as the end group.

Experimental Example 4

Results of PSA analysis

In order to specifically examine the diameters of the microparticles, analysis for Production Examples 1 to 11 was performed using a Microtrac particle size analyzer.
D10 to D90 are given in units of μm, and CV (%) was calculated by SD/mean* 100.
Span value was calculated by (D90-D 10)/D50 and indicates the uniformity of the particle size distribution.
The results of particle size analysis for the Production Examples are shown in Table 2 below.

[Table 2]

| | Production Example 1 | Production Example 2-1 | Production Example 2-2 | Production Example 3-1 | Production Example 3-2 | Production Example 4 | Production Example 5 | Production Example 6 |
|---|---|---|---|---|---|---|---|---|
| D10 | 71.27 | 78.36 | 75.90 | 78.52 | 74.66 | 77.40 | 73.46 | 68.69 |
| D20 | 75.74 | 81.69 | 78.09 | 82.33 | 76.88 | 80.20 | 76.68 | 72.62 |
| D30 | 78.91 | 84.63 | 79.98 | 85.61 | 78.76 | 82.77 | 79.27 | 75.42 |
| D40 | 81.87 | 87.40 | 81.84 | 88.63 | 80.56 | 85.33 | 81.74 | 77.77 |
| **D50** | **84.85** | **90.70** | **83.69** | **91.41** | **82.38** | **87.92** | **84.20** | **79.98** |
| D60 | 88.12 | 92.75 | 85.77 | 94.20 | 84.20 | 90.86 | 87.01 | 82.28 |
| D70 | 92.36 | 95.49 | 87.97 | 97.14 | 86.43 | 94.06 | 90.58 | 84.78 |
| D80 | 97.90 | 98.63 | 91.56 | 100.40 | 89.29 | 97.75 | 95.38 | 87.71 |
| D90 | 107.00 | 102.50 | 97.06 | 104.60 | 94.61 | 102.50 | 103.10 | 93.91 |
| Width | 26.29 | 19.46 | 16.19 | 21.08 | 15.01 | 20.32 | 22.51 | 18.46 |
| **Mean (MV)** | **90.85** | **93.29** | **85.26** | **96.05** | **83.67** | **89.51** | **87.22** | **81.87** |
| SD | 13.14 | 9.73 | 8.09 | 10.54 | 7.51 | 10.16 | 11.26 | 9.23 |
| Span value | 0.42 | 0.27 | 0.25 | 0.29 | 0.24 | 0.29 | 0.35 | 0.32 |

(continued)

| | Production Example 1 | Production Example 2-1 | Production Example 2-2 | Production Example 3-1 | Production Example 3-2 | Production Example 4 | Production Example 5 | Production Example 6 |
|---|---|---|---|---|---|---|---|---|
| CV(%) | 14.46 | 10.43 | 9.49 | 10.97 | 8.98 | 11.35 | 12.91 | 11.27 |

Referring to Table 2 above, it can be confirmed that the D50 values measured for the microparticles of the Production Examples of the present invention were 84.85 μm, 90.70 μm, 83.69 μm, 91.41 μm, 82.38 μm, 87.92 μm, 84.20 μm and 79.98 μm, respectively, and the CV values were 14.46%, 10.43%, 9.49%, 10.97%, 8.98%, 11.35%, 12.91% and 11.27%, respectively, suggesting that the microparticles had a uniform particle size distribution. In addition, it can be confirmed that the microparticles had average particle diameters (MV) of 90.85 μm, 93.29 μm, 85.26 μm, 96.05 μm, 86.67 μm, 89.51 μm, 87.22 μm and 81.87 μm, suggesting that they have a uniform particle size distribution within a range of 70 to 100 μm.

The results of particle size analysis for Production Examples 7-1 to 11 are shown in Table 3 below.

[Table 3]

| | Production Example 7-1 | Production Example 7-2 | Production Example 8 | Production Example 9-1 | Production Example 9-2 | Production Example 10 | Production Example 11 |
|---|---|---|---|---|---|---|---|
| D10 | 86.34 | 74.13 | 79 | 82.95 | 75.79 | 67.67 | 81.3 |
| D20 | 90.28 | 75.89 | 82.67 | 87.6 | 78.23 | 71.29 | 85.49 |
| D30 | 93.19 | 77.57 | 85.82 | 90.53 | 80.35 | 74.17 | 88.66 |
| D40 | 95.84 | 79.02 | 88.68 | 93.16 | 82.42 | 76.38 | 91.06 |
| **D50** | **98.5** | **80.46** | **91.23** | **95.64** | **84.5** | **78.52** | **93.32** |
| D60 | 101.3 | 81.96 | 93.78 | 98.26 | 86.88 | 80.61 | 95.52 |
| D70 | 104.4 | 83.47 | 96.38 | 101.1 | 89.86 | 82.88 | 97.89 |
| D80 | 108.9 | 85.24 | 99.29 | 104.4 | 93.93 | 85.45 | 100.3 |
| D90 | 115.3 | 87.28 | 102.9 | 111.4 | 99.9 | 88.98 | 103.6 |
| Width | 22.19 | 10.87 | 19.22 | 20.84 | 18.47 | 16.72 | 17.6 |
| **Mean(MV)** | **99.63** | **80.54** | **91.22** | **96.96** | **86.72** | **78.61** | **93.04** |
| SD | 11.09 | 5.43 | 9.61 | 10.42 | 9.23 | 8.36 | 8.8 |
| Span value | 0.29 | 0.16 | 0.26 | 0.30 | 0.29 | 0.27 | 0.24 |
| CV(%) | 11.13 | 6.74 | 10.53 | 10.75 | 10.64 | 10.63 | 9.46 |

Referring to Table 3 above, it can be confirmed that the D50 values measured for the microparticles of the Production Examples of the present invention were 98.5 μm, 80.46 μm, 91.23 μm, 95.64 μm, 84.5 μm, 78.52 μm and 93.32 μm, respectively, and the CV values were 11.13%, 6.74%, 10.53%, 10.75%, 10.64%, 10.63% and 9.46%, respectively, suggesting that the microparticles had a uniform particle size distribution. In addition, it can be confirmed that the microparticles had average particle diameters (MV) of 99.63 μm, 80.54 μm, 91.22 μm, 96.96 μm, 86.72 μm, 78.61 μm and 93.04 μm, suggesting that they had a uniform particle size distribution within a range of 70 to 100 μm.

Referring to the PSA analysis results in Tables 2 and 3 above, it can be confirmed that, when the prepared oil-phase solution contained 10 wt% of the polymer, the average particle size (MV) of the particles was 84.96 μm, and the standard deviation was 4.17, and when the oil-phase solution contained 15 wt% of the polymer, the average particle size (MV) of the particles was 94.97 μm, and the standard deviation was 3.17, suggesting that the average particle size (MV) of the particles increased as the concentration of the polymer in the oil-phase solution increased.

In addition, regarding the span value and CV(%) indicating the degree of particle size distribution, it can be seen that the particles according to each Production Example had a span value of 0.5 less and a CV(%) of 5 to 20%, suggesting that the particles produced according to the above-described production method had a very uniform particle size distribution.

Table 4 below shows the results of analyzing the particle size of the produced microparticles depending on the

content of the polymer in the oil phase solution.

[Table 4]

| Polymer Content | 5% | 10% | 15% | 20% |
|---|---|---|---|---|
| Polymer | Polylactide-co-glycolide (PLGA) | | | |
| ID | 101-OP30 | IC-4 | IC-17 | IC-16 |
| D10 | 46.00 | 66.38 | 81.67 | 90.66 |
| D20 | 48.33 | 69.40 | 85.89 | 93.63 |
| D30 | 50.36 | 71.99 | 88.95 | 96.30 |
| D40 | 52.23 | 74.35 | 91.29 | 98.86 |
| D50 | 53.97 | 76.41 | 93.47 | 101.50 |
| D60 | 55.72 | 78.49 | 95.60 | 104.40 |
| D70 | 57.55 | 80.56 | 97.90 | 108.20 |
| D80 | 59.59 | 82.93 | 100.20 | 112.80 |
| D90 | 62.47 | 85.73 | 103.40 | 118.90 |
| Width | 12.8 | 15.58 | 17.03 | 22.37 |
| **MV (μm)** | **57.98** | **76.27** | **93.14** | **103.30** |
| SD | 6.63 | 7.79 | 8.51 | 11.19 |
| Span value | 0.31 | 0.25 | 0.23 | 0.28 |
| CV(%) | 11.43 | 10.21 | 9.14 | 10.83 |

Referring to the PSA analysis results shown in Table 4 above, it was confirmed that, when the content of the polymer in the prepared oil-phase solution prepared using the same polymer (PLGA) was 5 wt%, the average particle size (MV) of the particles was 57.98 μm, and when the content of the polymer in the oil-phase solution was 10 wt%, the average particle size (MV) of the particles was 76.27 μm, and when the content of the polymer in the oil-phase solution was 15 wt%, the average particle size (MV) of the particles was 93.14 μm, and when the content of the polymer in the oil-phase solution was 20 wt%, the average particle size (MV) of the particles was 103.30 μm, suggesting that the average particle size (MV) of the particles increased as the content of the polymer in the oil-phase solution increased. Therefore, it can be seen that, when the same microchannels are used and the flow rate ratio of the oil phase and the water phase is kept constant under a condition in which dripping occurs, it is possible to produce microparticles having a desired particle size by changing the content (wt%) of the polymer in the oil-phase solution. Thereby, it is possible to control the drug release pattern depending on the size of microparticles, such as initial burst suppression, and the content of the polymer can be used to set the drug retention period.

Experimental Example 5

Results of SEM analysis

In order to specifically examine the appearance of the microparticles, the analysis of Preparation Examples 1 to 11 was performed using a scanning electron microscope (SEM).
The SEM analysis results for Preparation Examples 1 to 11 are shown in FIGS. 3 to 18.
Referring to the SEM photographs, it was confirmed that the produced microparticles had a uniform particle size, a smooth surface, and a perfectly spherical shape.

Experimental Example 6

Pharmacokinetic analysis results

The pharmacokinetic evaluation of Example 1 was performed.
Example 1 was a 6-month or longer sustained-release formulation, and examination was made as to whether the

formulation maintained the efficacy of moxidectin by releasing moxidectin sustainably over 6 month after injection.

Through the microparticles of the present invention, moxidectin was administered by SC injection to a total of five beagle dogs at a dose of 0.4 mg/kg.

The results of analyzing the plasma concentration of moxidectin are shown in Table 5 below.

[Table 5]

| Example 1 | | |
|---|---|---|
| Time (hr) | Average | STDEV |
| 0 | 0.00 | 0.00 |
| 48 | 0.31 | 0.05 |
| 72 | 0.71 | 0.15 |
| 168 | 2.27 | 0.34 |
| 240 | 3.55 | 0.56 |
| 336 | 4.44 | 0.49 |
| 408 | 5.95 | 0.95 |
| 504 | 8.86 | 1.97 |
| **600** | **15.56** | **3.05** |
| 672 | 14.81 | 3.94 |
| 720 | 11.74 | 3.13 |
| 1008 | 5.82 | 2.29 |
| 1344 | 4.89 | 1.54 |
| 1680 | 6.31 | 3.38 |
| 1848 | 8.43 | 4.81 |
| 2016 | 9.05 | 4.33 |
| **2184** | **11.55** | **6.45** |
| 2352 | 8.50 | 4.53 |
| 2520 | 10.13 | 4.65 |
| 2688 | 6.13 | 2.45 |
| 2856 | 6.19 | 2.96 |
| 3024 | 4.51 | 2.74 |
| 3360 | 3.21 | 2.51 |
| 3696 | 2.62 | 2.21 |
| 4032 | 1.34 | 1.46 |
| 4320 | 0.76 | 0.86 |

Referring to the experimental results in Table 5 above, it was confirmed that, in the plasma concentration of moxidectin, there was no excessive release of moxidectin at an initial stage, and a first Cmax appeared around 25 days after injection. After 25 days, the plasma concentration of moxidectin decreased constantly, and after 56 days, the plasma concentration of moxidectin increased. Then, a second Cmax appeared around 91 days. Accordingly, it can be confirmed that the value calculated by the following Equation 1, which represents the plasma concentration of moxidectin measured after mixing the microparticles containing moxidectin with a suspending solution to obtain an injectable formulation and administering the injectable formulation to a plurality of beagle dogs, was 1.35:

[Equation 1]

$$C_{max\text{-}peak\ n}/C_{max\text{—}peak\ n+1}$$

wherein

$C_{max\text{-}peak\ n}$ is an $n^{th}$ Cmax value after administration of the injectable formulation, and
$C_{max\text{-}peak\ n+1}$ is an $n+1^{st}$ Cmax value measured when the plasma concentration of moxidectin increases again after the $n^{th}$ Cmax value.

This plasma concentration pattern of moxidectin is for the injectable formulation prepared by producing microparticles containing two types of biodegradable polymers, respectively, and mixing these microparticles together. The degradation rate of the biodegradable polymer differs depending on the viscosity and molecular weight of the biodegradable polymer and a combination of the biodegradable polymer and moxidectin, and thus the plasma concentration and release pattern described above appear.

In addition, the results of analyzing the pharmacokinetics of Example 1 are shown in Table 6 below.

[Table 6]

| Example 1 | Average | STDEV |
|---|---|---|
| AUClast (ng·hr/ml) | 25084.66 | 6983.41 |
| AUCinf (ng·hr/ml) | 26028.26 | 7034.78 |
| Cmax (ng/ml) | 16.73 | 4.32 |
| Tmax (hr) | 916.80 | 708.39 |
| T1/2 | 466.89 | 121.18 |

## Experimental Example 7

Pharmacokinetic analysis results

Pharmacokinetic evaluation for Example 2 was performed.

The experiment was conducted in the same manner as in Example 1, except that the microparticles of Production Example 1 and the microparticles of Production Example 2-1 were mixed together at a weight ratio of 1:1.

[Table 7]

| Example 2 | | |
|---|---|---|
| Time (hr) | Average | STDEV |
| 0 | 0.00 | 0.00 |
| 24 | 0.75 | 0.24 |
| 72 | 1.32 | 0.45 |
| 168 | 2.93 | 0.85 |
| 240 | 4.68 | 1.88 |
| 336 | 7.01 | 3.45 |
| 408 | 9.45 | 3.70 |
| **504** | **12.79** | **5.64** |
| 600 | 11.93 | 4.72 |
| 672 | 11.69 | 5.51 |
| 720 | 11.21 | 3.83 |

(continued)

| Example 2 | | |
|---|---|---|
| Time (hr) | Average | STDEV |
| 1008 | 630 | 2.72 |
| 1344 | 4.55 | 1.61 |
| **1680** | **5.72** | **2.11** |
| 1848 | 5.31 | 1.49 |
| 2016 | 518 | 1.76 |
| 2184 | 4.57 | 1.67 |
| 2352 | 3.47 | 2.72 |
| 2520 | 3.54 | 2.45 |
| 2688 | 2.70 | 204 |
| 2856 | 1.78 | 1.51 |
| 3024 | 1.50 | 1.27 |
| 3360 | 1.23 | 1.19 |
| 3696 | 1.00 | 0.00 |
| 4032 | 0.68 | 0.00 |
| 4320 | 0.47 | 0.00 |

Referring to the experimental results shown in Table 7 above, it was confirmed that, in the plasma concentration of moxidectin, there was excessive release of moxidectin at an initial stage, and a first Cmax appeared around 21 days after injection. After 21 days, the plasma concentration of moxidectin decreased constantly, and after 56 days, the plasma concentration of moxidectin increased. Then, a second Cmax appeared around 70 days. Thus, it can be confirmed that the value calculated by Equation 1 was 2.24.

In addition, 24 hours after injection, the plasma concentration of moxidectin was 0.75 ng/ml, and the standard deviation of the plasma concentration between the beagle dogs subjected to the experiment was 0.24.

The results of analyzing the pharmacokinetics of Example 2 are shown in Table 8 below.

[Table 8]

| Example 2 | Average | STDEV |
|---|---|---|
| AUClast (ng·hr/ml) | 17701.36 | 3301.50 |
| AUCinf (ng·hr/ml) | 18717.96 | 3736.92 |
| Cmax (ng/ml) | 13.66 | 4.61 |
| Tmax (hr) | 576.00 | 124.71 |

Experimental Example 8

Pharmacokinetic analysis results

Pharmacokinetic analysis of Example 3 was performed.

The experiment was conducted in the same manner as in Example 1, except that 50% of the dose administered in Example 1 was used. The microparticles of Production Example 1 and the microparticles of Production Example 2-1 were mixed together at a weight ratio of 1:2 in the same manner as in Example 1.

[Table 9]

| Example 3 | | |
|---|---|---|
| Time (days) | Mean | SD |
| 0 | 0.00 | 0.00 |
| 7 | 0.44 | 0.40 |
| 14 | 1.19 | 0.46 |
| 21 | 1.96 | 0.81 |
| 28 | 2.84 | 0.92 |
| **35** | **3.36** | **1.16** |
| 49 | 1.90 | 0.66 |
| 63 | 1.61 | 0.85 |
| 77 | 2.37 | 0.78 |
| 84 | 2.39 | 0.35 |
| 91 | 2.54 | 0.83 |
| 98 | 4.71 | 1.62 |
| **105** | **6.00** | **1.65** |
| 112 | 5.99 | 2.57 |
| 126 | 5.40 | 1.46 |
| 140 | 3.17 | 1.34 |
| 154 | 1.60 | 0.95 |
| 168 | 1.06 | 1.04 |
| 182 | 0.50 | 0.91 |

Referring to the experimental results shown in Table 9 above, it was confirmed that, in the plasma concentration of moxidectin, there was no excessive release of moxidectin at an initial stage, and a first Cmax appeared around 35 days after injection. After 35 days, the plasma concentration of moxidectin decreased constantly, and after 77 days, the plasma concentration of moxidectin increased. Then, a second Cmax appeared around 105 days. Accordingly, it was confirmed that the value calculated by Equation 1 was 0.56.

Experimental Example 9

Pharmacokinetic analysis results

An *in vivo* experiment was conducted to confirm the reproducibility upon administration of the same test drug by comparing the plasma concentration of moxidectin between subjects to which the drug was administered.

For comparison, currently commercially available ProHeart SR-12 was administered to three beagle dogs, and the plasma concentration of moxidectin was measured for 180 days.

As the microparticles of the present invention, Example 3 was administered to a total of 10 beagles at a moxidectin dose of 0.2 mg/kg, and the plasma concentration of moxidectin was measured for 180 days.

The experimental results are shown in Tables 10 and 11 below and FIG. 19.

[Table 10]

| ProHeart SR-12 (moxidectin dose: 0.5 mg/kg) | | | | | |
|---|---|---|---|---|---|
| Time (days) | 1 | 2 | 3 | Mean | SD |
| 0 | 0.00 | 0.00 | 0.00 | 0.00 | 0.00 |
| 7 | 11.96 | 10.30 | 12.02 | 11.43 | 0.98 |
| 14 | 31.22 | 12.20 | 9.50 | 17.64 | 11.84 |

(continued)

| ProHeart SR-12 (moxidectin dose: 0.5 mg/kg) | | | | | |
|---|---|---|---|---|---|
| Time (days) | 1 | 2 | 3 | Mean | SD |
| 21 | 28.13 | 13.36 | 6.36 | 15.95 | 11.11 |
| 28 | 26.40 | 12.38 | 4.59 | 14.46 | 11.05 |
| 35 | 27.77 | 10.28 | 3.20 | 13.75 | 12.65 |
| 49 | 20.42 | 7.83 | 1.62 | 9.96 | 9.58 |
| 63 | 18.33 | 6.25 | 0.84 | 8.47 | 8.95 |
| 77 | 16.52 | 3.71 | 0.72 | 6.98 | 8.39 |
| 84 | 15.29 | 3.71 | 0.86 | 6.62 | 7.64 |
| 91 | 13.88 | 2.70 | 0.76 | 5.78 | 7.08 |
| 98 | 11.15 | 1.89 | 0.73 | 4.59 | 5.71 |
| 105 | 12.89 | 1.72 | 0.77 | 5.13 | 6.74 |
| 112 | 9.22 | 1.70 | 0.75 | 3.89 | 4.64 |
| 126 | 6.35 | 1.28 | 0.46 | 2.70 | 3.19 |
| 140 | 5.59 | 1.30 | 0.61 | 2.50 | 2.70 |
| 154 | 7.06 | 1.52 | 0.75 | 3.11 | 3.44 |
| 168 | 4.35 | 1.39 | 0.62 | 2.12 | 1.97 |
| 180 | 3.8 | 1.34 | 0.57 | 1.90 | 1.69 |
| (unit: ng/ml) | | | | | |

[Table 11]

| Example 3 | | | | | | | | | | | | |
|---|---|---|---|---|---|---|---|---|---|---|---|---|
| Time (days) | 1 | 2 | 3 | 4 | 5 | 6 | 7 | 8 | 9 | 10 | Mean | SD |
| 0 | 0.00 | 0.00 | 0.00 | 0.00 | 0.00 | 0.00 | 0.00 | 0.00 | 0.00 | 0.00 | 0.00 | 0.00 |
| 7 | 0.87 | 0.55 | 0.33 | 0.92 | 0.85 | 0.69 | 0.43 | 0.73 | 0.78 | 0.42 | 0.66 | 0.21 |
| 14 | 1.36 | 0.98 | 0.36 | 1.72 | 2.05 | 1.29 | 0.60 | 1.74 | 1.45 | 0.86 | 1.24 | 0.54 |
| 21 | 3.29 | 1.59 | 1.31 | 3.03 | 3.68 | 2.15 | 1.30 | 3.11 | 2.17 | 1.07 | 2.27 | 0.95 |
| 28 | 3.44 | 3.22 | 1.77 | 3.82 | 4.75 | 3.49 | 2.24 | 3.60 | 3.38 | 1.51 | 3.12 | 0.99 |
| 35 | 3.06 | 2.97 | 1.79 | 2.32 | 4.75 | 4.54 | 2.01 | 3.15 | 4.56 | 2.54 | 3.17 | 1.09 |
| 49 | 1.19 | 0.86 | 0.97 | 1.52 | 2.88 | 2.84 | 1.30 | 2.36 | 1.54 | 1.48 | 1.69 | 0.74 |
| 63 | 2.34 | 2.32 | 0.66 | 1.88 | 3.42 | 2.73 | 0.64 | 2.20 | 1.49 | 1.01 | 1.87 | 0.92 |
| 77 | 2.65 | 1.77 | 0.92 | 2.31 | 3.70 | 3.20 | 1.61 | 3.19 | 2.18 | 1.68 | 2.32 | 0.87 |
| 84 | 1.97 | 1.08 | 1.18 | 2.80 | 4.46 | 2.83 | 2.05 | 2.36 | 2.64 | 2.05 | 2.34 | 0.96 |
| 91 | 1.70 | 0.95 | 1.25 | 2.81 | 4.49 | 3.70 | 1.90 | 3.00 | 2.41 | 1.67 | 2.39 | 1.12 |
| 98 | 3.78 | 1.89 | 1.85 | 4.94 | 5.08 | 7.17 | 3.45 | 5.06 | 4.80 | 3.06 | 4.11 | 1.63 |
| 105 | 3.34 | 1.98 | 2.29 | 5.52 | 6.24 | 8.50 | 3.96 | 6.34 | 5.80 | 5.42 | 4.94 | 2.03 |
| 112 | 2.96 | 3.26 | 2.11 | 4.97 | 7.15 | 9.84 | 3.47 | 7.33 | 4.78 | 4.55 | 5.04 | 2.39 |
| 126 | 4.74 | 2.72 | 3.31 | 3.10 | 7.75 | 7.62 | 5.32 | 5.35 | 5.17 | 3.52 | 4.86 | 1.78 |
| 140 | 1.67 | 1.10 | 2.59 | 0.90 | 4.48 | 5.41 | 2.69 | 2.82 | 3.11 | 1.84 | 2.66 | 1.43 |
| 154 | - | 0.69 | 1.32 | - | 2.74 | 3.21 | 0.93 | 1.53 | 1.52 | 0.83 | 1.60 | 0.91 |

(continued)

| Example 3 | | | | | | | | | | | | |
|---|---|---|---|---|---|---|---|---|---|---|---|---|
| Time (days) | 1 | 2 | 3 | 4 | 5 | 6 | 7 | 8 | 9 | 10 | Mean | SD |
| 168 | - | - | 1.01 | - | 1.75 | 2.87 | - | 1.01 | 0.75 | 0.68 | 1.35 | 0.84 |
| 180 | - | - | 0.57 | - | 1.10 | 1.88 | - | 0.60 | - | - | 1.04 | 0.61 |
| (unit: ng/ml) | | | | | | | | | | | | |

Referring to the experimental results shown in Tables 10 and 11 above, it can be confirmed that commercially available ProHeart SR-12 showed a large difference in plasma concentration between the beagle dogs, whereas, in the case of Example 3 of the present invention, the standard deviation of the measured plasma concentration of moxidectin between the 10 beagle dogs was 0.2 to 2.39, which was very insignificant.

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

## Industrial Applicability

The present invention relates to microparticles containing moxidectin and a sustained-release injectable composition containing the same.

## Claims

1. Microparticles containing moxidectin and a biodegradable polymer, wherein the biodegradable polymer has an intrinsic viscosity of 0.1 dl/g to 1 dl/g, and the microparticles have an average diameter of 60 to 110 μm.

2. The microparticles of claim 1, wherein the microparticles are spherical in shape, and moxidectin is distributed uniformly in the microparticles.

3. The microparticles of claim 1, wherein the microparticles have a coefficient of variation (CV) of 5% to 20%.

4. The microparticles of claim 1, wherein the microparticles contain the biodegradable and moxidectin at a weight ratio of 2:1 to 12:1.

5. The microparticles of claim 1, wherein the microparticles release moxidectin sustainably over 3 months or more.

6. The microparticles of claim 1, wherein the biodegradable polymer is selected from the group consisting of polylactide (PLA), polylactide-co-glycolide (PLGA), polyphosphazene, polyiminocarbonate, polyphosphoester, polyanhydride, polyorthoester, polycaprolactone, polyhydroxyvalerate, polyhydroxybutyrate, polyamino acids, and mixtures thereof.

7. The microparticles of claim 1, wherein the microparticles have a value of 0.3 to 3 as calculated by the following Equation 1, which represents a plasma concentration of moxidectin measured after mixing the microparticles containing moxidectin with a suspending solution to obtain an injectable formulation and administering the injectable formulation to a plurality of beagle dogs:

[Equation 1]

$$C_{max\text{-}peak\ n}/C_{max\text{—}peak\ n+1}$$

wherein

$C_{max\text{-}peak\ n}$ is an $n^{th}$ Cmax value after administration of the injectable formulation, and
$C_{max\text{-}peak\ n+1}$ is an $n+1^{st}$ Cmax value measured when the plasma concentration of moxidectin increases again

after the $n^{th}$ Cmax value.

**8.** The microparticles of claim 7, wherein the plasma concentration of moxidectin is measured after administering the injectable formulation to the plurality of beagle dogs at a moxidectin dose of 0.2 mg/kg, and a standard deviation of the plasma concentration of moxidectin between the beagle dogs to which the injectable formulation has been administered is 0.01 to 10.

**9.** A moxidectin-containing sustained-release injectable composition containing:

microparticles containing moxidectin according to claim 1; and
a suspending solution.

FIG. 1

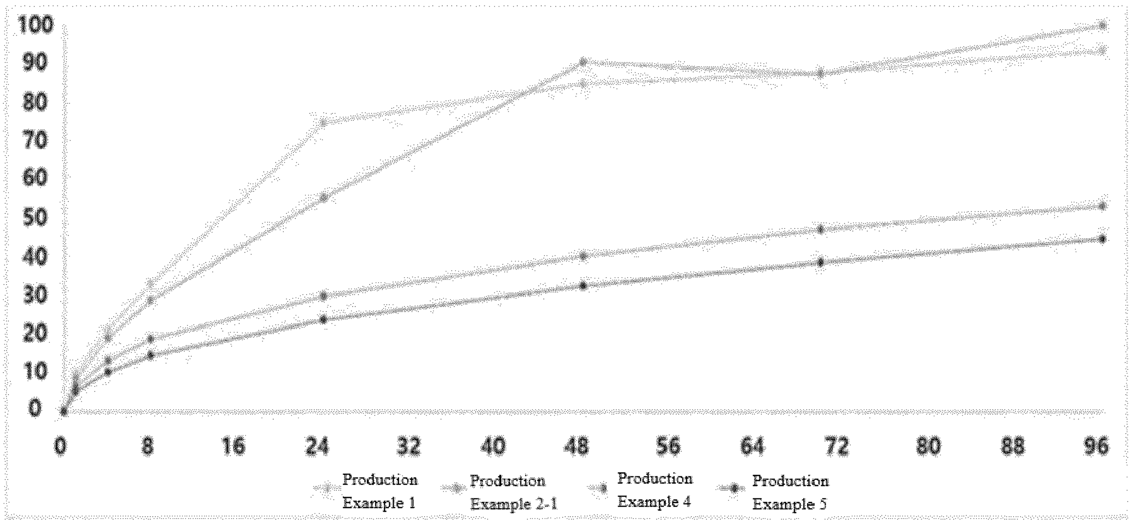
100
90
80
70
60
50
40
30
20
10
0
0
8
16
24
32
40
48
56
64
72
80
88
96
Production Example 1
Production Example 2-1
Production Example 4
Production Example 5

FIG. 2

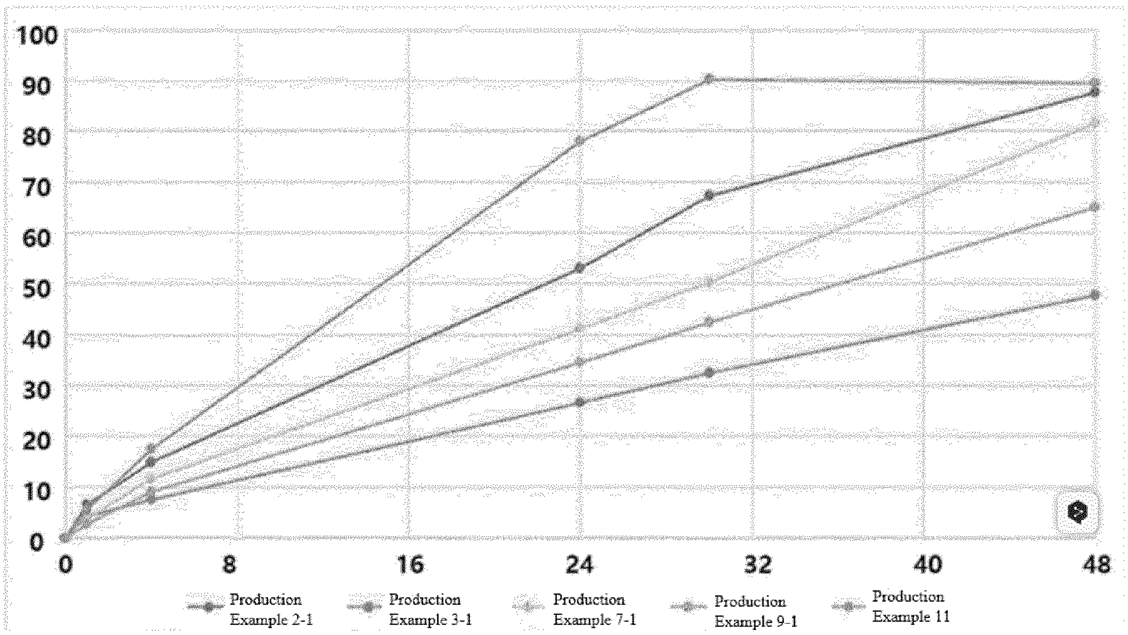
100
90
80
70
60
50
40
30
20
10
0
0
8
16
24
32
40
48
Production Example 2-1
Production Example 3-1
Production Example 7-1
Production Example 9-1
Production Example 11

FIG. 3

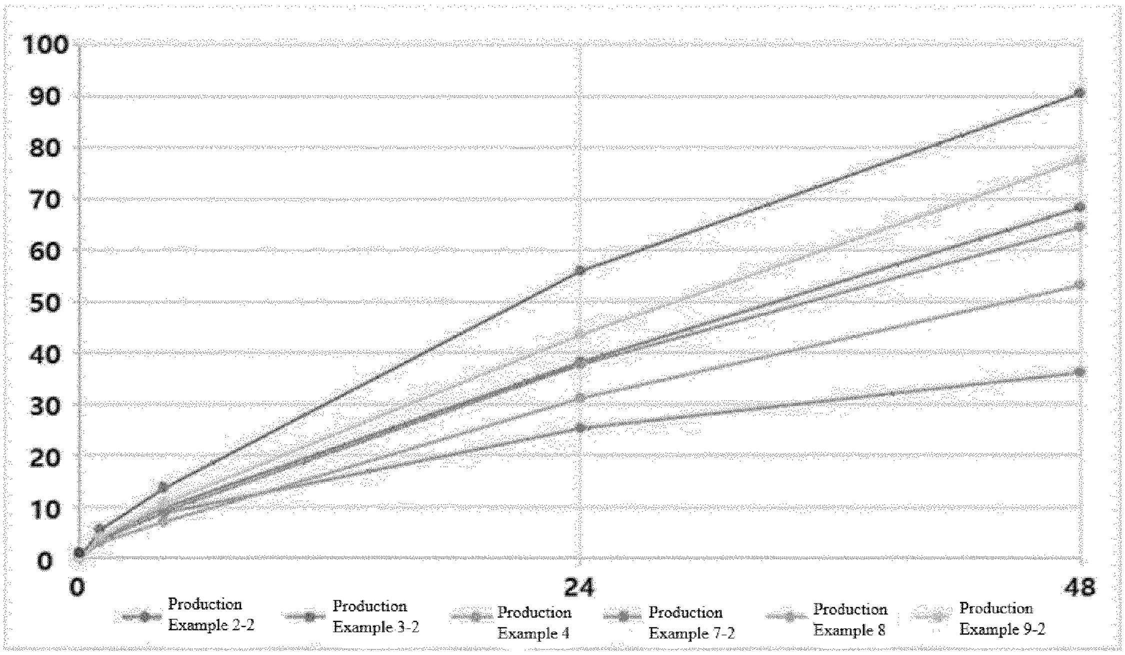
100
90
80
70
60
50
40
30
20
10
0
0
24
48
Production Example 2-2
Production Example 3-2
Production Example 4
Production Example 7-2
Production Example 8
Production Example 9-2

FIG. 4

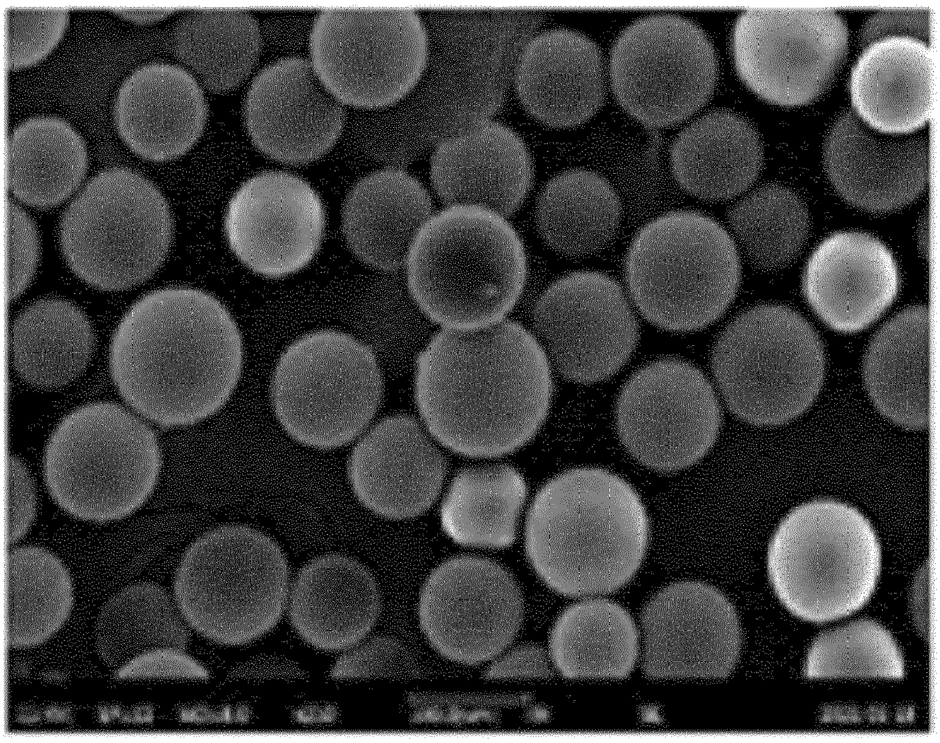

FIG. 5

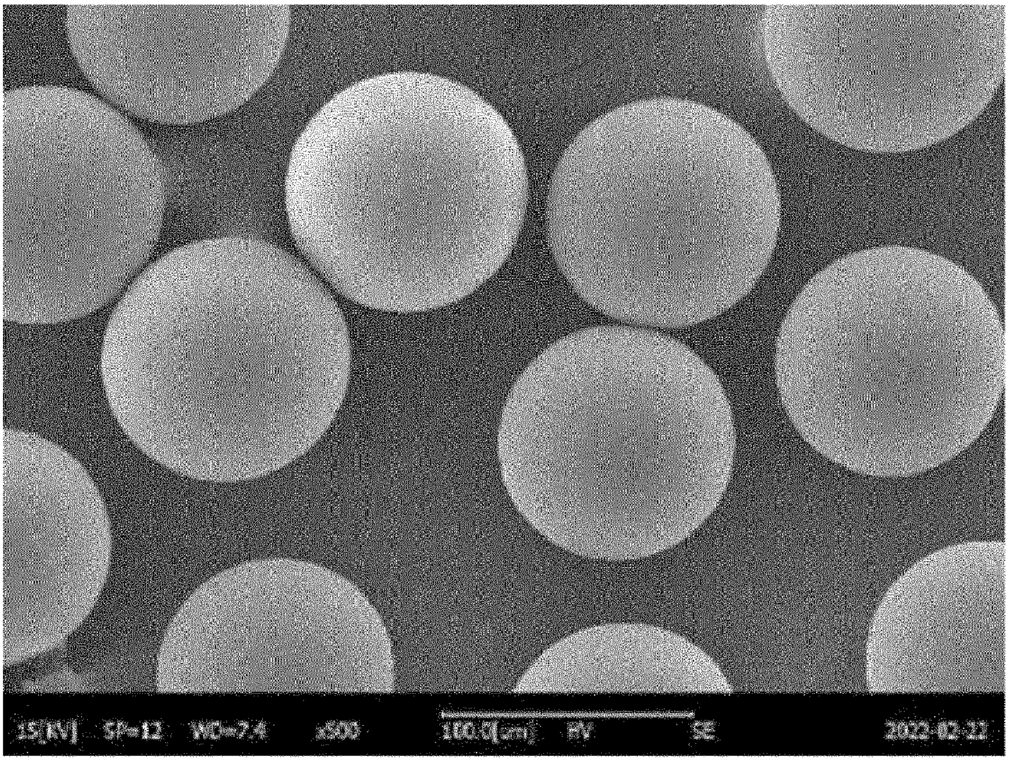
15[KV] SP=12 WD=7.4 x500
100.0[μm] HV SE
2022-02-22

FIG. 6

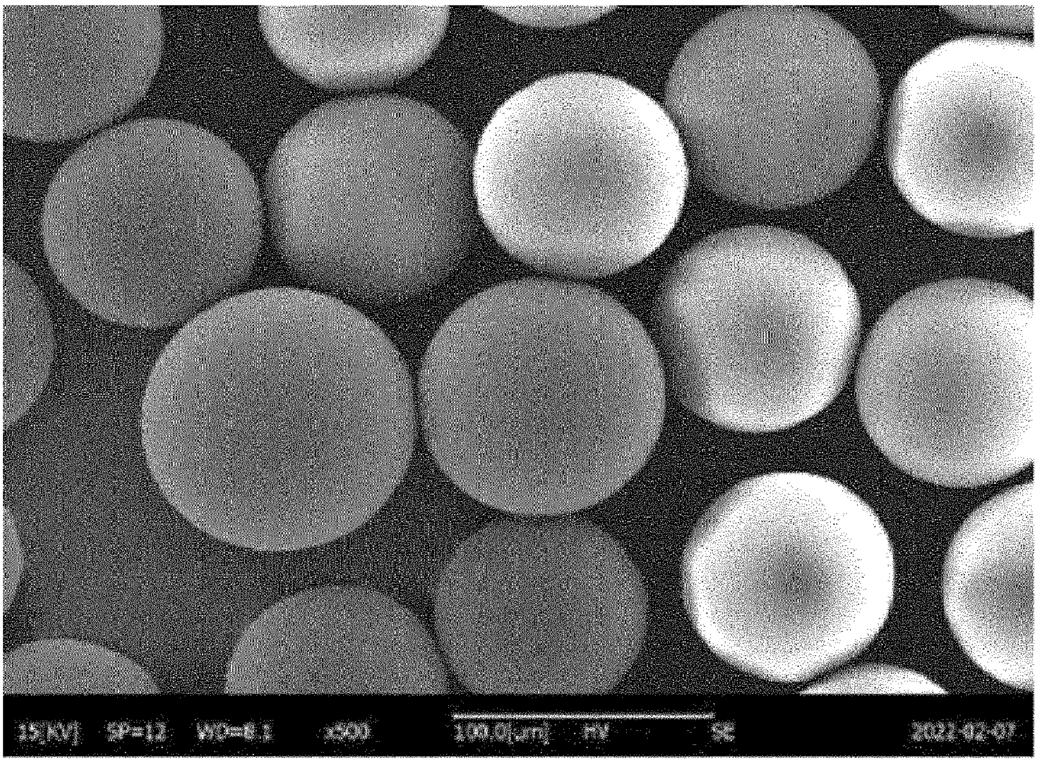
15[KV] SP=12 WD=8.1 x500
100.0[μm] HV SE
2022-02-07

FIG. 7

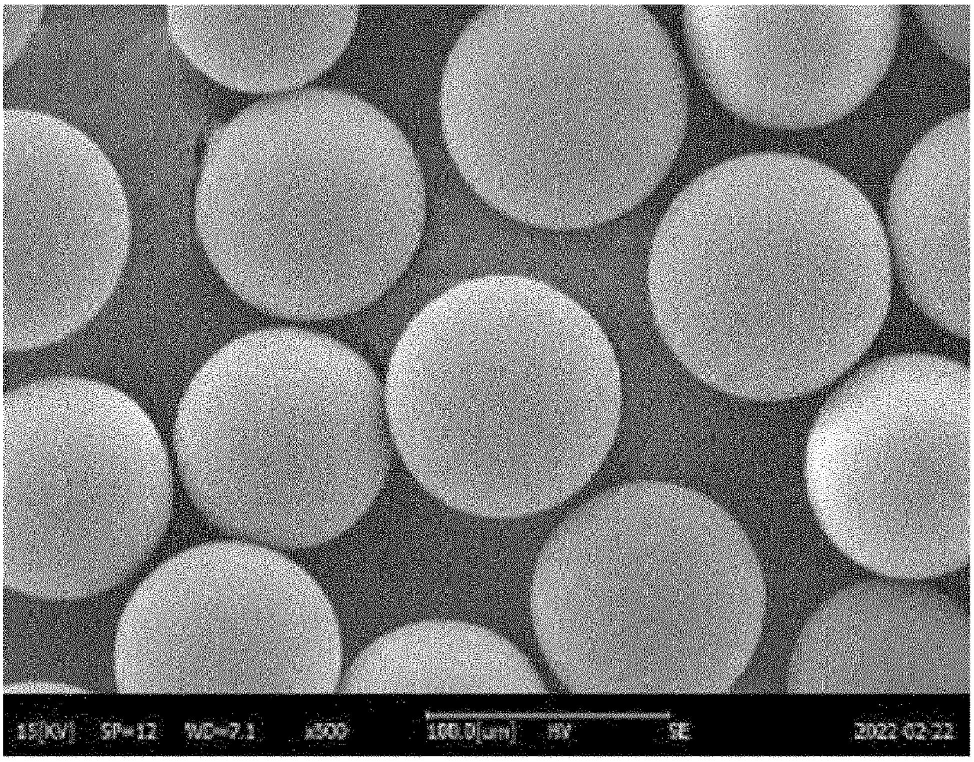
15[KV] SP=12 WD=7.1 x500
100.0[um] HV SE
2022-02-22

FIG. 8

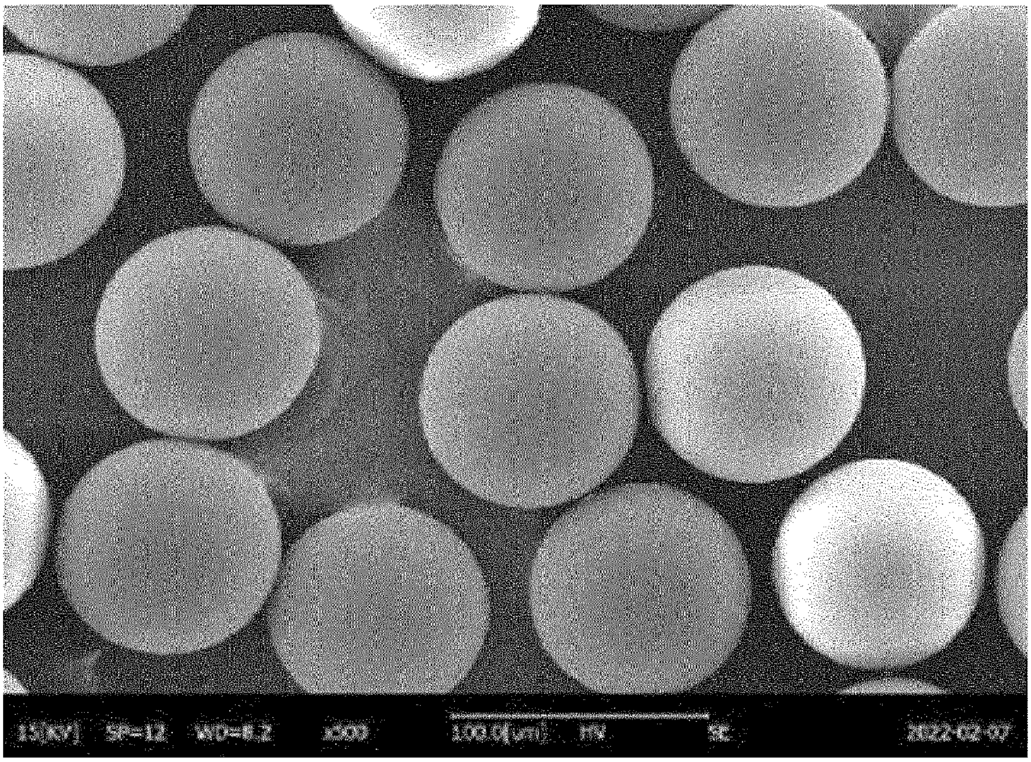
15[KV] SP=12 WD=8.2 x500
100.0[um] HV SE
2022-02-07

FIG. 9

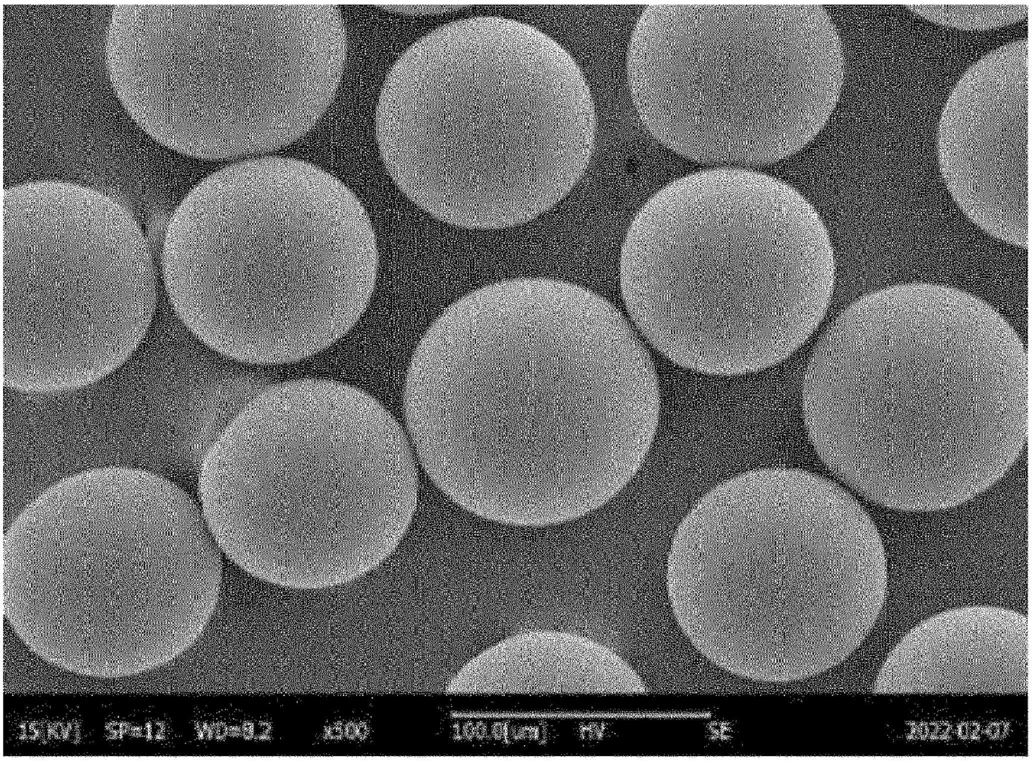


FIG. 10

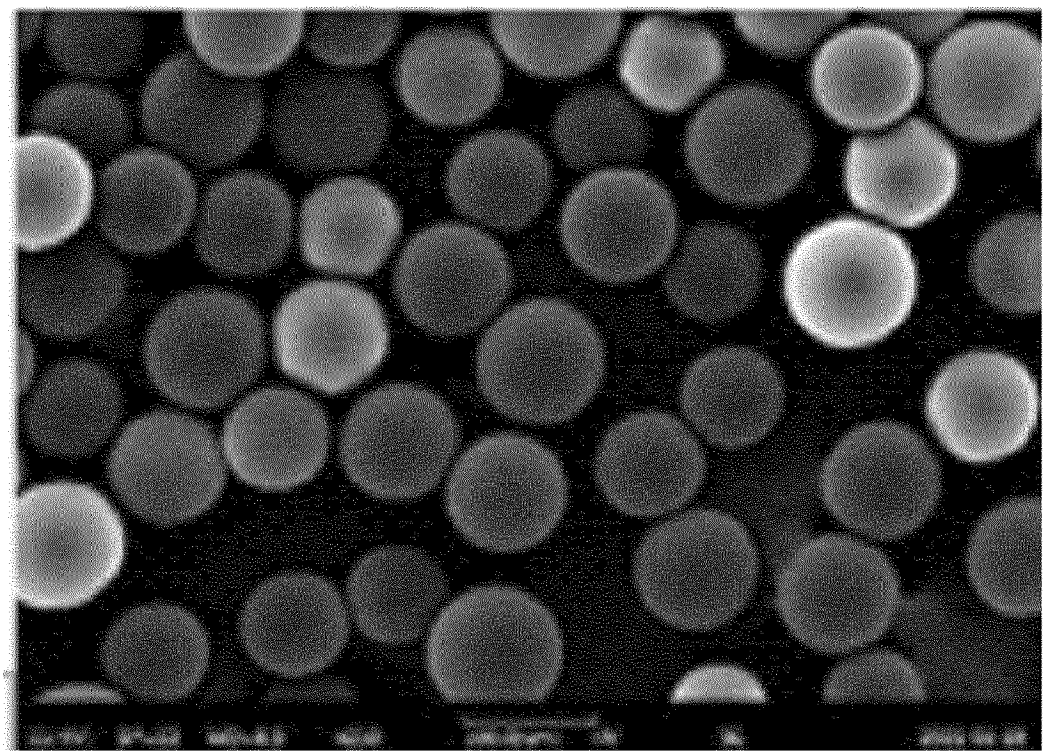

**FIG. 11**

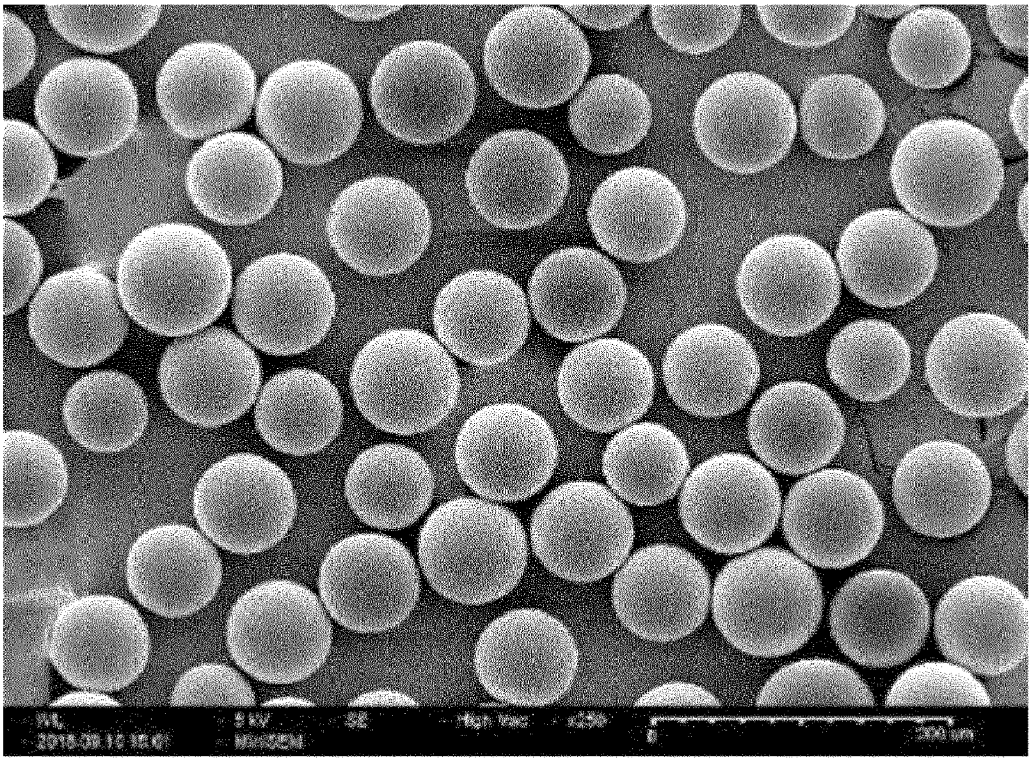

**FIG. 12**

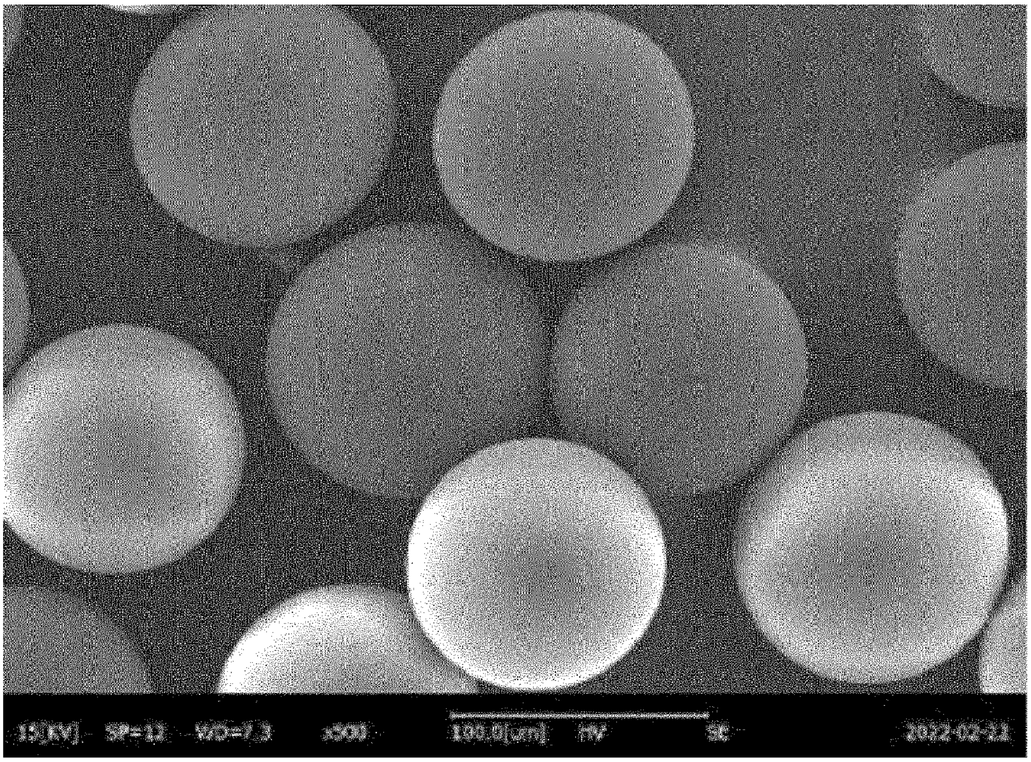

**FIG. 13**

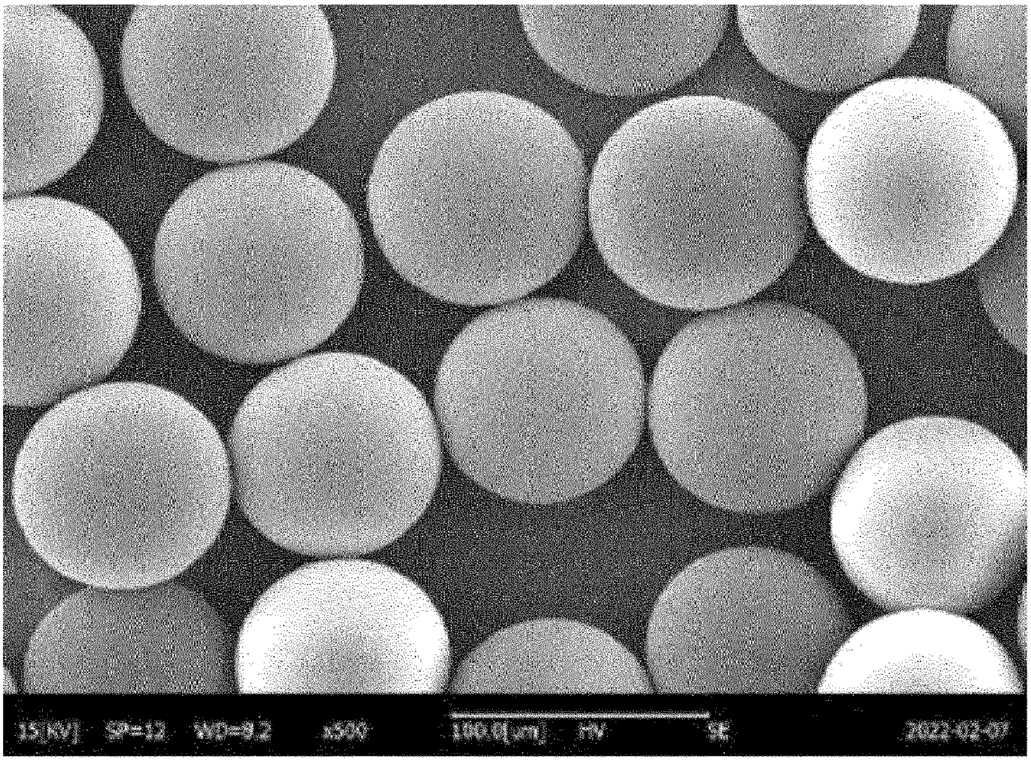
15[KV] SP=12 WD=8.2 x500
100.0[μm] HV SE
2022-02-07

**FIG. 14**

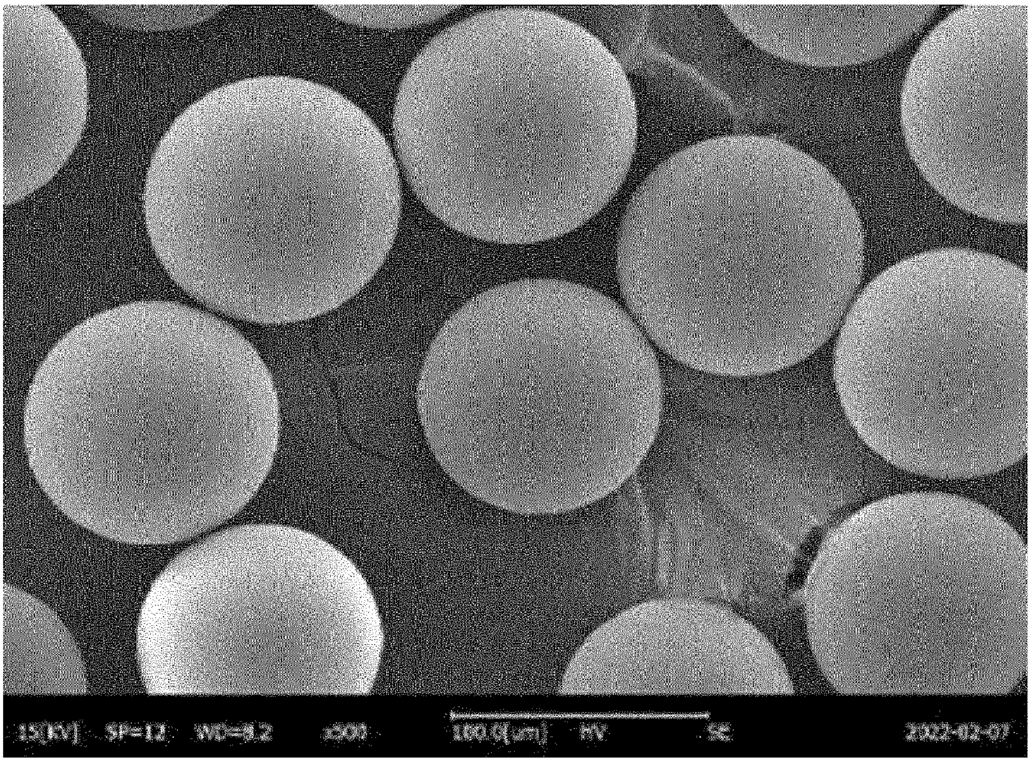
15[KV] SP=12 WD=8.2 x500
100.0[μm] HV SE
2022-02-07

FIG. 15

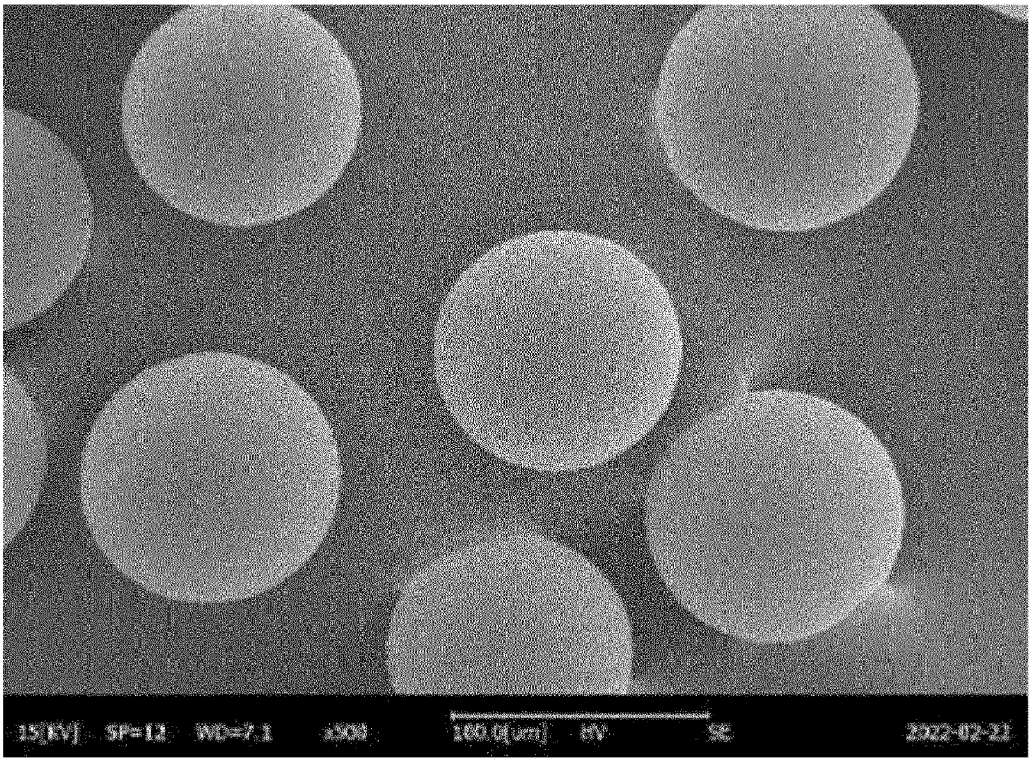
15[KV] SP=12 WD=7.1 x500
100.0[um] HV SE
2022-02-22

FIG. 16

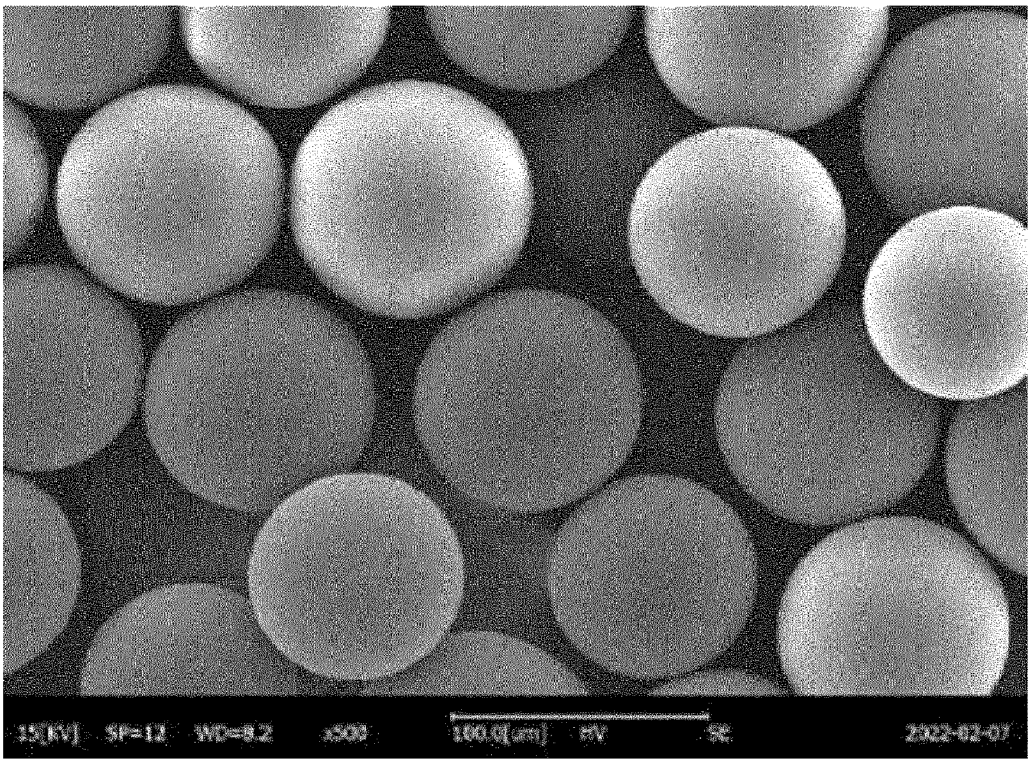
15[KV] SP=12 WD=8.2 x500
100.0[um] HV SE
2022-02-07

FIG. 17

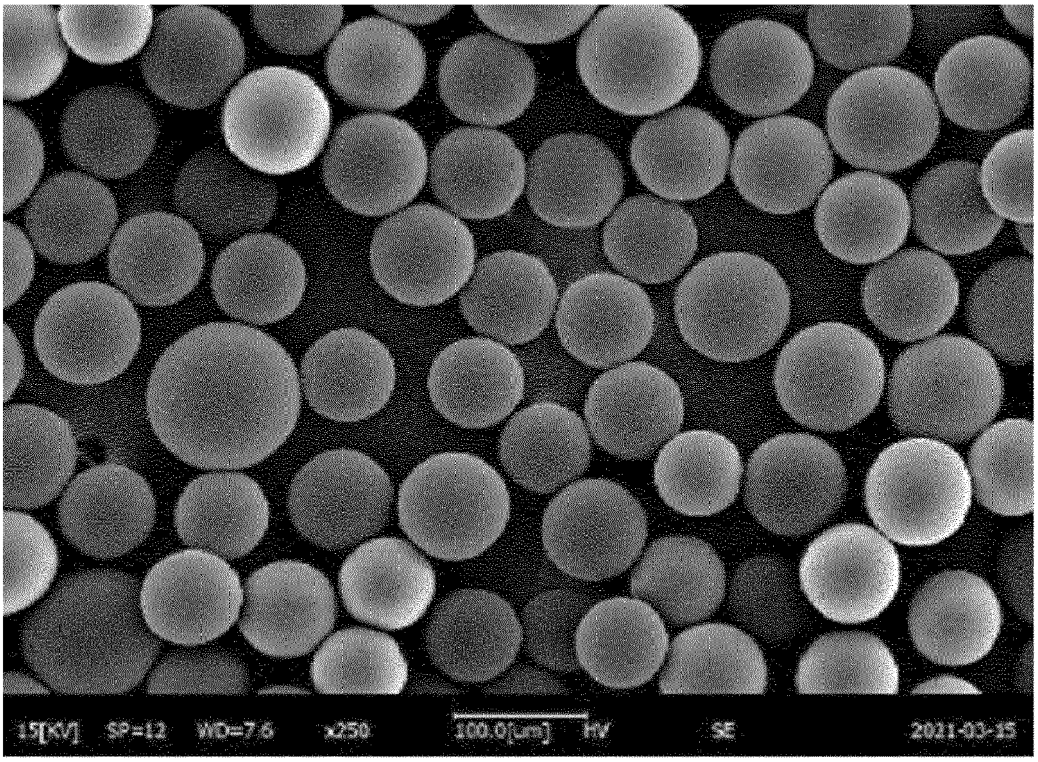
15[KV] SP=12 WD=7.6 x250 100.0[um] HV SE 2021-03-15

FIG. 18

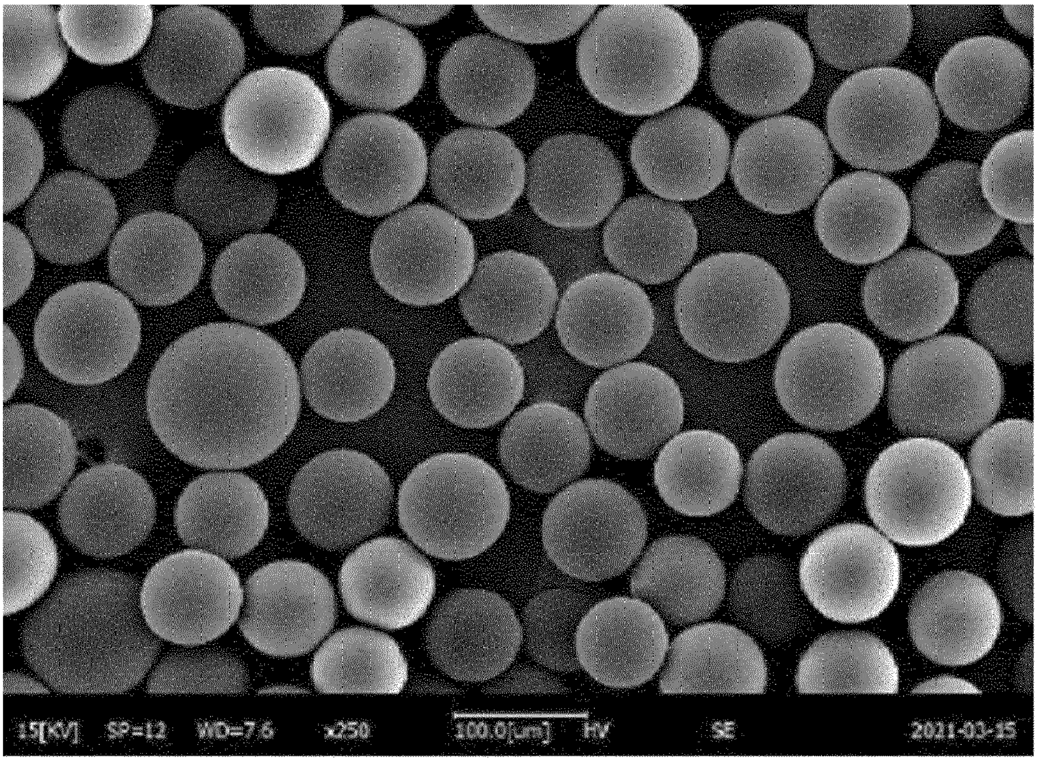
15[KV] SP=12 WD=7.6 x250 100.0[um] HV SE 2021-03-15

FIG. 19

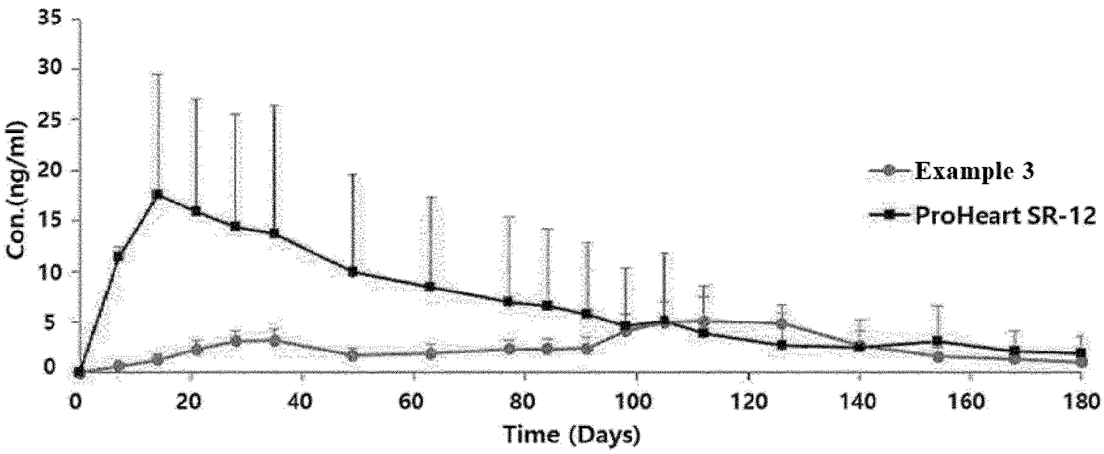
35
30
25
20
15
10
5
0
Con.(ng/ml)
0
20
40
60
80
100
120
140
160
180
Time (Days)
Example 3
ProHeart SR-12

**INTERNATIONAL SEARCH REPORT**

International application No.
**PCT/KR2022/005968**

**A. CLASSIFICATION OF SUBJECT MATTER**

**A61K 9/16**(2006.01)i; **A61K 31/365**(2006.01)i; **A61K 9/00**(2006.01)i; **A61P 33/10**(2006.01)i

According to International Patent Classification (IPC) or to both national classification and IPC

**B. FIELDS SEARCHED**

Minimum documentation searched (classification system followed by classification symbols)

A61K 9/16(2006.01); A01N 25/00(2006.01); A01N 43/04(2006.01); A61K 31/58(2006.01); A61K 47/34(2006.01); A61K 9/20(2006.01); A61K 9/22(2006.01); A61K 9/50(2006.01); B01J 13/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 목시덱틴(moxidectin), 생분해성 고분자(biodegradable polymer), 마이크로입자(microparticle), 고유점도(intrinsic viscosity), 직경(diameter)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2019-0027302 A (INVENTAGE LAB INC. et al.) 14 March 2019 (2019-03-14)<br>See paragraphs [0031]-[0032]; and claims 1-4 and 8. | 1-9 |
| Y | KR 10-2012-0115302 A (EVONIK DEGUSSA CORPORATION) 17 October 2012 (2012-10-17)<br>See paragraph [0078]; examples 1-7; and claim 1. | 1-9 |
| A | US 2007-0042013 A1 (SOLL, Mark D. et al.) 22 February 2007 (2007-02-22)<br>See entire document. | 1-9 |
| A | KR 10-2022-0032301 A (INVENTAGE LAB INC.) 15 March 2022 (2022-03-15)<br>See entire document. | 1-9 |
| A | KR 10-1350680 B1 (WARSAW ORTHOPEDIC, INC. et al.) 10 February 2014 (2014-02-10)<br>See entire document. | 1-9 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 January 2023** | **06 January 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2022/005968**

| Patent document cited in search report | Publication date (day/month/year) | Patent family member(s) | Publication date (day/month/year) |
|---|---|---|---|
| KR 10-2019-0027302 A | 14 March 2019 | CN 110381924 A | 25 October 2019 |
| | | EP 3733164 A1 | 04 November 2020 |
| | | EP 3733164 A4 | 24 November 2021 |
| | | JP 2021-509662 A | 01 April 2021 |
| | | JP 7040818 B2 | 23 March 2022 |
| | | KR 10-2019-0027349 A | 14 March 2019 |
| | | KR 10-2101969 B1 | 22 April 2020 |
| | | US 2020-0375902 A1 | 03 December 2020 |
| | | WO 2019-050259 A1 | 14 March 2019 |
| KR 10-2012-0115302 A | 17 October 2012 | CN 102985175 A | 20 March 2013 |
| | | CN 102985175 B | 09 March 2016 |
| | | EP 2516053 A2 | 31 October 2012 |
| | | EP 2516053 A4 | 21 August 2013 |
| | | EP 2516053 B1 | 20 November 2019 |
| | | JP 2013-515059 A | 02 May 2013 |
| | | JP 2015-037792 A | 26 February 2015 |
| | | JP 5791629 B2 | 07 October 2015 |
| | | JP 5984903 B2 | 06 September 2016 |
| | | KR 10-1862416 B1 | 29 May 2018 |
| | | US 2011-0204533 A1 | 25 August 2011 |
| | | US 9486416 B2 | 08 November 2016 |
| | | WO 2011-087689 A2 | 21 July 2011 |
| | | WO 2011-087689 A3 | 17 November 2011 |
| US 2007-0042013 A1 | 22 February 2007 | EP 1962794 A2 | 03 September 2008 |
| | | EP 1962794 B1 | 14 November 2018 |
| | | EP 3479818 A1 | 08 May 2019 |
| | | JP 2009-504778 A | 05 February 2009 |
| | | JP 5132560 B2 | 30 January 2013 |
| | | KR 10-1351644 B1 | 14 January 2014 |
| | | KR 10-2008-0038235 A | 02 May 2008 |
| | | US 8362086 B2 | 29 January 2013 |
| | | WO 2007-024719 A2 | 01 March 2007 |
| | | WO 2007-024719 A3 | 09 August 2007 |
| KR 10-2022-0032301 A | 15 March 2022 | KR 10-2022-0032509 A | 15 March 2022 |
| | | KR 10-2022-0032510 A | 15 March 2022 |
| | | KR 10-2022-0032511 A | 15 March 2022 |
| | | US 2022-0249452 A1 | 11 August 2022 |
| | | US 2022-0339105 A1 | 27 October 2022 |
| | | WO 2022-050783 A1 | 10 March 2022 |
| KR 10-1350680 B1 | 10 February 2014 | CN 101835466 A | 15 September 2010 |
| | | CN 104997722 A | 28 October 2015 |
| | | EP 2219599 A2 | 25 August 2010 |
| | | JP 2011-518182 A | 23 June 2011 |
| | | JP 5341172 B2 | 13 November 2013 |
| | | KR 10-2010-0055512 A | 26 May 2010 |
| | | US 2009-0264490 A1 | 22 October 2009 |
| | | US 2015-0148396 A1 | 28 May 2015 |
| | | US 2015-0148397 A1 | 28 May 2015 |
| | | US 8946277 B2 | 03 February 2015 |
| | | US 9585872 B2 | 07 March 2017 |

Form PCT/ISA/210 (patent family annex) (July 2019)

**INTERNATIONAL SEARCH REPORT**
**Information on patent family members**

International application No.
**PCT/KR2022/005968**

| Patent document cited in search report | Publication date (day/month/year) | Patent family member(s) | Publication date (day/month/year) |
|---|---|---|---|
| | | US 9763917 B2 | 19 September 2017 |
| | | WO 2009-129460 A2 | 22 October 2009 |
| | | WO 2009-129460 A3 | 25 February 2010 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020060005472 A1